# EUROPEAN PATENT SPECIFICATION

(11) **EP 2 440 568 B1**
(45) Date of publication and mention of the grant of the patent: **25.02.2015**
(21) Application number: 10784029.0
(22) Date of filing: 02.06.2010
(51) Int. Cl.: C07K 1/10, C12N 9/00

(54) **CROSS-LINKING OF SUPEROXIDE DISMUTASE MONOMERS**
VERNETZUNG VON SUPEROXID-DISMUTASE-MONOMEREN
RÉTICULATION DE MONOMÈRES DE SUPEROXYDE DISMUTASE

(30) Priority: 02.06.2009 US 183286 P
(43) Date of publication of application: 18.04.2012
(73) Proprietor: Brandeis University, Waltham, MA 02453-2728 (US)
(72) Inventor: AGAR, Jeffrey, N., Newton MA 02459 (US)
(74) Representative: Trueman, Lucy Petra
(86) International application number: PCT/US2010/037104
(87) International publication number: WO 2010/141613

(56) References cited:
- EP-A1- 1 803 808
- WO-A2-2005/077040
- DE-A1- 3 628 508
- US-B1- 6 565 842
- HONGBIN LIU ET AL: "Copper(2+) Binding to the Surface Residue Cysteine 111 of His46Arg Human Copper-Zinc Superoxide Dismutase, a Familial Amyotrophic Lateral Sclerosis Mutant +", BIOCHEMISTRY, vol. 39, no. 28, 1 July 2000 (2000-07-01), pages 8125-8132, XP55045716, ISSN: 0006-2960, DOI: 10.1021/bi000846f
- M. COZZOLINO ET AL: "Cysteine 111 Affects Aggregation and Cytotoxicity of Mutant Cu,Zn-superoxide Dismutase Associated with Familial Amyotrophic Lateral Sclerosis", JOURNAL OF BIOLOGICAL CHEMISTRY, vol. 283, no. 2, 11 January 2008 (2008-01-11), pages 866-874, XP55045715, ISSN: 0021-9258, DOI: 10.1074/jbc.M705657200
- PARGE ET AL: "Atomic structures of wild-type and thermostable mutant recombinant human Cu,Zn superoxide dismutase.", PROCEEDINGS OF THE NATIONAL ACADEMY OF SCIENCES, vol. 89, no. 13, 1 July 1992 (1992-07-01), pages 6109-6113, XP55045717, ISSN: 0027-8424
- LEPOCK ET AL: "Contribution of conformational stability and reversibility of unfolding to the increased thermostability of human and bovine superoxide dismutase mutated at free cysteines.", JOURNAL OF BIOLOGICAL CHEMISTRY, vol. 265, no. 35, 1 December 1990 (1990-12-01), pages 21612-21618, XP55045720, ISSN: 0021-9258
- RACHEL L. REDLER ET AL: "Glutathionylation at Cys-111 Induces Dissociation of Wild Type and FALS Mutant SOD1 Dimers", BIOCHEMISTRY, vol. 50, no. 32, 16 August 2011 (2011-08-16), pages 7057-7066, XP55045723, ISSN: 0006-2960, DOI: 10.1021/bi200614y
- PERRY J J P ET AL: "The structural biochemistry of the superoxide dismutases", BIOCHIMICA ET BIOPHYSICA ACTA (BBA) - PROTEINS & PROTEOMICS, ELSEVIER, NETHERLANDS, vol. 1804, no. 2, 1 February 2010 (2010-02-01), pages 245-262, XP026867188, ISSN: 1570-9639, DOI: 10.1016/J.BBAPAP.2009.11.004 [retrieved on 2009-11-13]
- J. R. AUCLAIR ET AL: "Strategies for stabilizing superoxide dismutase (SOD1), the protein destabilized in the most common form of familial amyotrophic lateral sclerosis", PROCEEDINGS OF THE NATIONAL ACADEMY OF SCIENCES, vol. 107, no. 50, 14 December 2010 (2010-12-14), pages 21394-21399, XP055003713, ISSN: 0027-8424, DOI: 10.1073/pnas.1015463107
- FURUKAWA ET AL.: 'Disulfide cross-linked protein represents a significant fraction of ALS-associated Cu, Zn-superoxide dismutase aggregates in spinal cords of model mice' PNAS vol. 103, no. 18, 02 May 2006, pages 7148 - 7153, XP008149319
- HALLEWELL ET AL.: 'Thermostabilization of recombinant human and bovine CuZn superoxide dismutases by replacement of free cysteines' BBRC vol. 181, no. 1, 1991, pages 474 - 480, XP027115049
- SAU ET AL.: 'Mutation of SOD1 in ALS: a gain of a loss of function' HUMAN MOL. GENET. vol. 16, no. 13, 2007, pages 1604 - 1618, XP008149320
- WITAN ET AL.: 'Heterodimer formation of wild-type and amyotrophic lateral sclerosis-causing mutant Cu/Zn-superoxide dismutase induces toxicity independent of protein aggregation' HUMAN MOL. GENET. vol. 17, no. 10, 2008, pages 1373 - 1385, XP008149204

## Description

### BACKGROUND OF THE INVENTION

Innovative approaches are needed to combat neurodegenerative disease, among the most serious of which is Amyotrophic Lateral Sclerosis (ALS), a disorder characterized by the death of both upper and lower motor neurons and by 3-5 years median survival post diagnosis. The only FDA-approved drug for the treatment of ALS, Riluzole, has at best, moderate effect on patient survival and quality of life (1-3). Of the greater than fifty clinical trials for ALS involving everything from anti-inflammatory compounds to metals to antioxidants, no effective drug has been identified. Notably, despite the growing evidence that aggregation and loss of protein stability are part of fALS etiology, not a single aggregation inhibitor or pharmacological chaperone has been brought to clinical trial. The only clinical trial that we are aware of targeting SOD1 specifically is a small, ongoing Phase I trial involving antisense oligonucleotides. Interestingly, ALS clinical trials have by and large involved the same strategies and many of the same compounds used in Parkinson's trials, although it is likely they do not share common disease pathways.

While the causes of sporadic neurodegenerative diseases remain a mystery, mutations causing familial forms of many of these diseases (e.g., Alzheimer, Parkinson, and ALS) are known. For example, mutations in the gene encoding Cu/Zn Superoxide Dismutase (SOD1) are responsible for ∼20% of the familial ALS cases (fALS) and 2% of all ALS. Two such mutations are G93A, which maintains wild-type like enzymatic activity, and the metal-deficient G85R, which is essentially inactive. Post-translational modifications of proteins involved in familial diseases have been invoked in the etiology of the corresponding sporadic diseases, for example alpha-synuclein and Parkin modification in Parkinson, a beta and tau modification in Alzheimer, and TDP43 and SOD 1 modification in ALS. The hope, therefore, is that strategies for treating familial diseases may translate to at least a subset of sporadic diseases.

Both dominant inheritance of mutant SOD1 and lack of symptoms in knock-out mice suggest a "gain of toxic function" as opposed to a loss of function. Aggregation propensity and loss of stability of SOD1 are synergistic risk factors for fALS patient disease severity, and it has been suggested that a common property of fALS variants in vitro and in vivo is their propensity to aggregate. A prevailing hypothesis for the mechanism of the toxicity of fALS-SOD1 variants involves dimer destabilization and dissociation into monomers, which then nucleate the formation of higher order aggregates. Indeed, variant proteins such the G85R SOD1 used in this study, are found as monomers in vivo. A number of modifications, including loss of Cu or Zn, cleavage of the native, intramolecular disulfide, oxidation, and fALS-associated mutation, predispose SOD1 to dimer dissociation. X-ray crystal structures of both A4V, and to a lesser extent I113T; yeast two hybrid analysis of H46R, A4V, and H48Q; dissociation of G85R, G93R, E100G, and I113T by chaotrophs; and molecular dynamics simulations are all consistent with this hypothesis. In addition to the hypothesis that dimer destabilization causes aggregation, another hypothesis for aggregation is that newly translated fALS SOD1 causing variants never dimerize due to lack of intrasubunit disulfide formation, metal-deficiency, etc. resulting in unstable monomer. Consistent with this, stabilization of the SOD1 dimer interface by tethering subunits, or through the use of small molecules, may prevent protein aggregation.

EP1803808 A1 provides a lecithinized superoxide dismutase (PC-SOD) composition disclosed as useful as a drug material and a process for its production.

US4818698 A (corresponding to DE3628508 A1) has as its object the provision of novel h-SOD (human-origin SOD) analogous polypeptides to be useful as medicines.

Thiol-disulfide exchange may be explored as one representative approach to tethering a stable SOD1 dimer. Thiol-disulfide exchange is well-suited for investigation as a therapeutic strategy and is in fact known to occur in vivo on SOD1 Cys111, resulting in SOD1 binding the tripeptide glutathione (GSH). These suggest that the Cys111 residues at the dimer interface of SOD1 are a potential target for therapeutics. Moreover, human SOD1 Cys111 is one of at least two residues (the other being SOD1 W32) that modulate the toxicity of fALS mutations. For example, the C111S mutant increases SOD1 stability and reverses the cytotoxicity and aggregation of the fALS SOD1 mutations C6F, C146R, G93A, A4V, and H46R. Chemical modification of Cys111 by alkylation also increases SOD1 stability.

### SUMMARY OF THE INVENTION

One aspect of the invention is a stabilized superoxide dismutase analogue, wherein said analogue has a tertiary structure and comprises a first SOD1 monomer, a second SOD1 monomer, and a crosslinker; said crosslinker comprises a spacer arm with length from 3 Å to 15 Å; wherein said first SOD1 monomer comprises a first cysteine at a first position corresponding to position 111 of wild-type human SOD1, and said second SOD1 monomer comprises a second cysteine at a second position corresponding to position 111 of wild-type human SOD1; and the first cysteine is covalently connected to the second cysteine by the crosslinker. Another aspect of the invention is a method of producing a stabilized superoxide dismutase (SOD1) analogue, comprising the step of reacting a first SOD1 monomer, a second SOD1 monomer, and a cross-linker, thereby forming said analogue. An effective representative site for this cross-linking is the 111 position on each monomer. The invention also includes a method of producing the SOD1 analogue, wherein a first cysteine of a first SOD1 monomer and a second cysteine of a second SOD1 monomer are connected by a cross-linker. Another aspect of the invention is a stabilized SOD1 analogue of the invention for use in treatment or prophylaxis for a neurodegenerative disease. A representative neurological disease for which this invention may have significant efficacy is amyotrophic lateral sclerosis. Also disclosed is a pharmaceutical composition, comprising an SOD1 analogue, and a pharmaceutically acceptable carrier.

### BRIEF DESCRIPTION OF THE FIGURES

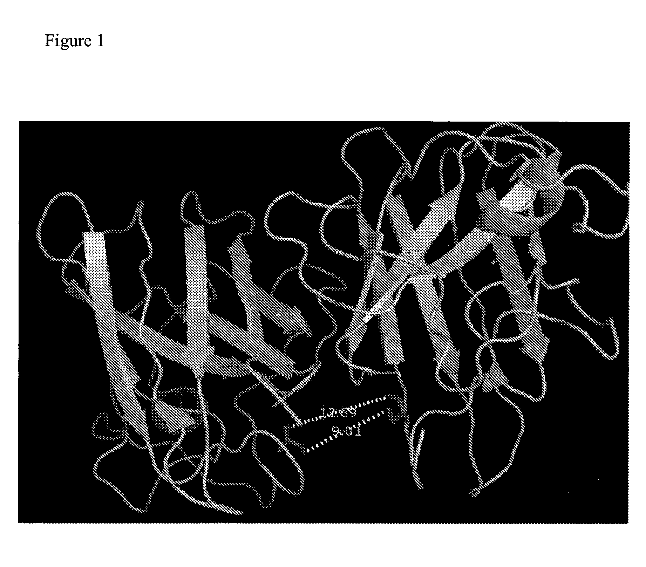

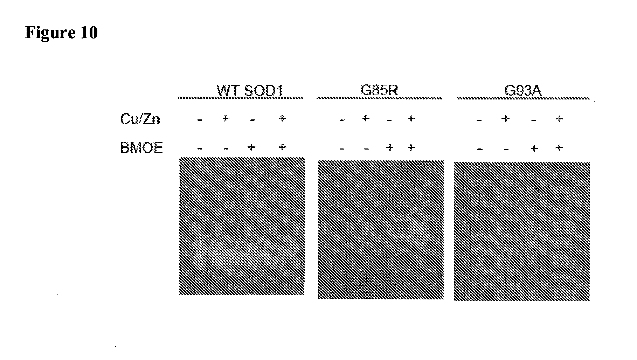

**Figure 1** shows the site of Cysteine 111 intersubunit cross-linking in SOD1. Cysteine 111 residues on opposing SOD1 monomers are shown at each end.

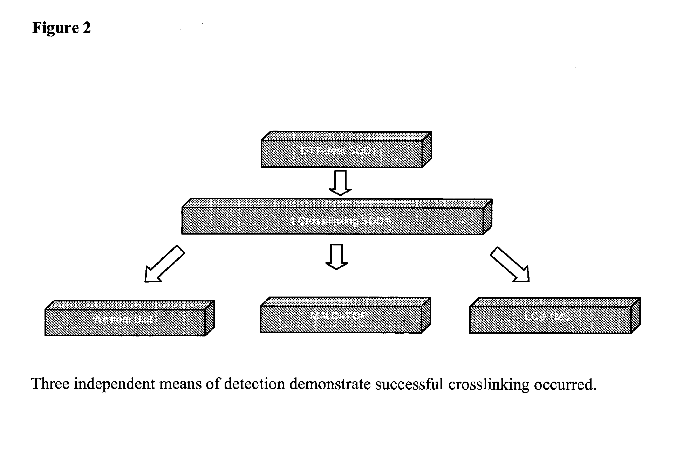

**Figure 2** shows a block diagram summarizing certain methods used in cross-linking experiments.

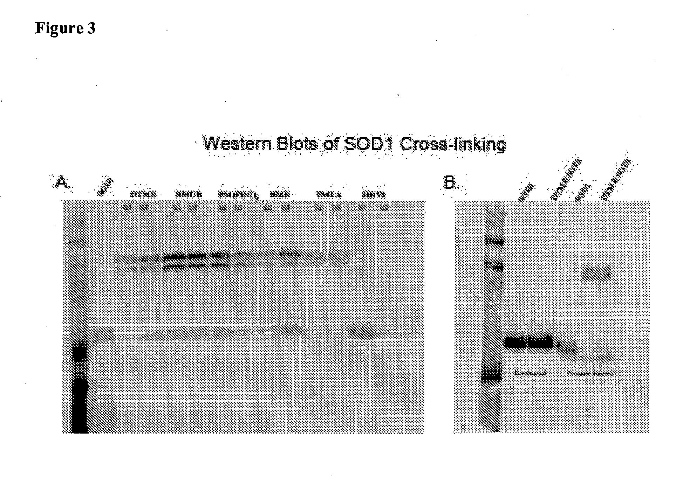

**Figure 3** shows the results of Western blot analysis of certain SOD1 cross-linking experiments. (A) Cross-linking with different maleimide and vinylsulfone cross-linkers. SOD1 was cross-linked with a 1:1 or 1:3 concentration of cross-linker to protein. Each of the maleimide cross-linkers cross-linked SOD1 resulting in dimer formation. TMEA, a trifunctional cross-linker, appeared to be the most efficient cross-linker. HBVS, a vinylsulfone, did not appear to cross-link SOD1. (B) DTME cross-linking of SOD1. SOD1 was cross-linked with DTME, which contains a disulfide bond in its linker region, and run on an SDS PAGE in the presence or absence of reducing agent. The reducing agent reduced the cross-linker returning the dimer to its monomeric form.

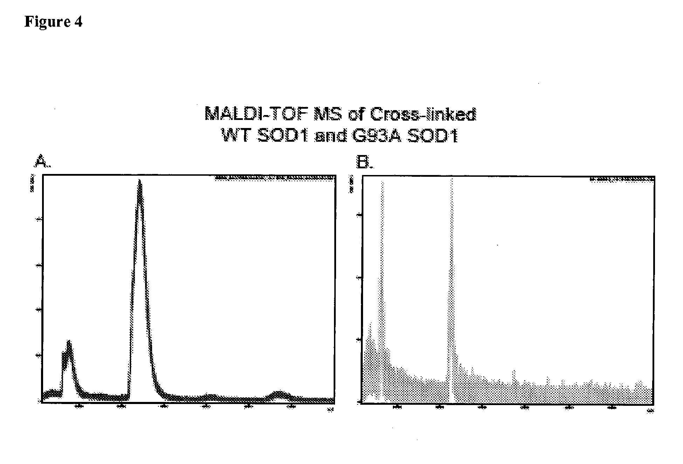

**Figure 4** shows the results of MALDI-TOF MS analysis of cross-linked SOD1. (A) Cross-linking of wild-type SOD1. (B) Cross-linking of G93A. Both WT and G93A SOD1 are cross-linked using maleimide cross-linkers as seen by the increase in dimer observed in the MALDI-TOF analysis of cross-linked SOD1.

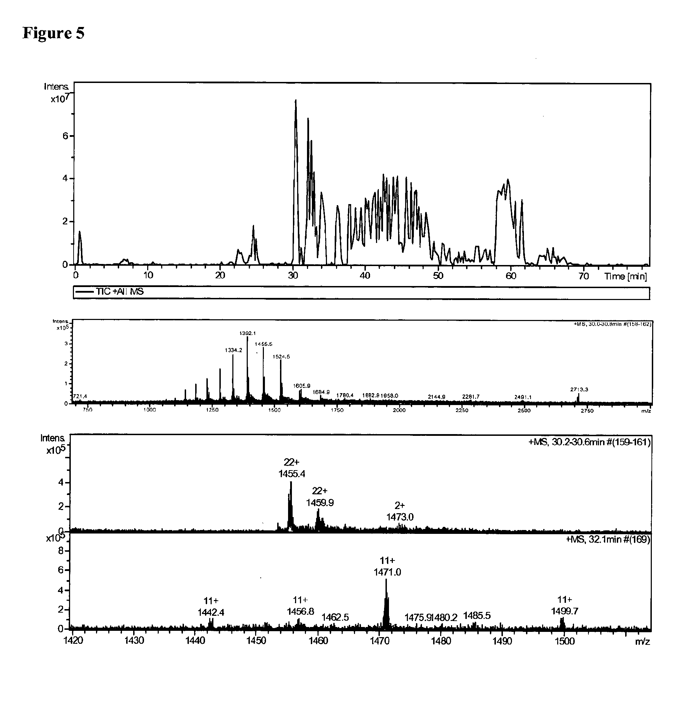

**Figure 5** shows LC-FTMS analysis of cross-linked SOD1. LC-FTMS Spectra of cross-linked G93A SOD1. (A) The total ion chromatogram (TIC) for the liquid chromatography run; the dimer elutes at approximately 30 minutes. (B) The unprocessed spectra of dimeric G93A related to 32.1 minutes in the TIC. (C) The G93A dimer at 1455.4 modified by one cross-linker. (D) Funnel skimmer dissociation of the G93A cross-linked dimer. The m/z of 1442.4 corresponds to the G93A monomer, m/z of 1456.8 corresponds to the G93A monomer modified by the cleaved cross-linker, m/z of 1471.0 corresponds to the G93A monomer modified by one cross-linker, and m/z of 1499.7 corresponds to G93A modified by two cross-linkers.

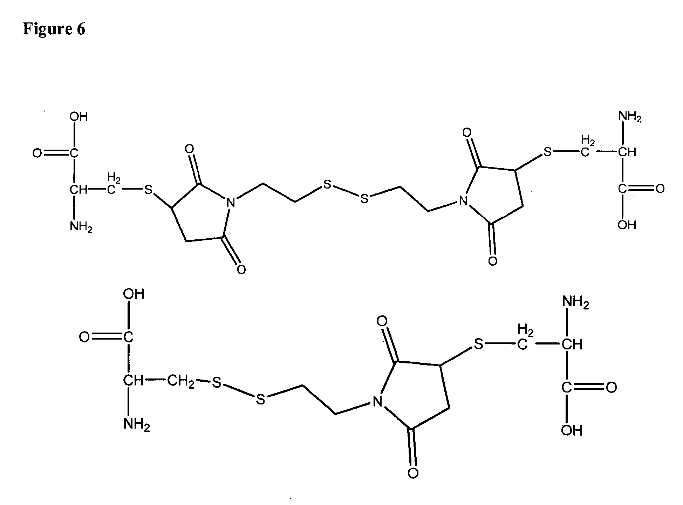

**Figure 6****:** depicts the reaction products from cross-linking of SOD1 dimers using DTME as a homobifunctional and heterobifunctional cross-linker. Reaction products from crosslinking of SOD1 dimers using DTME as a homobifunctional (top) and heterobifunctional (bottom) crosslinker. The upper structure uses two maleimide initiated reactions, whereas the bottom structure uses maleimide and disulfide exchange.

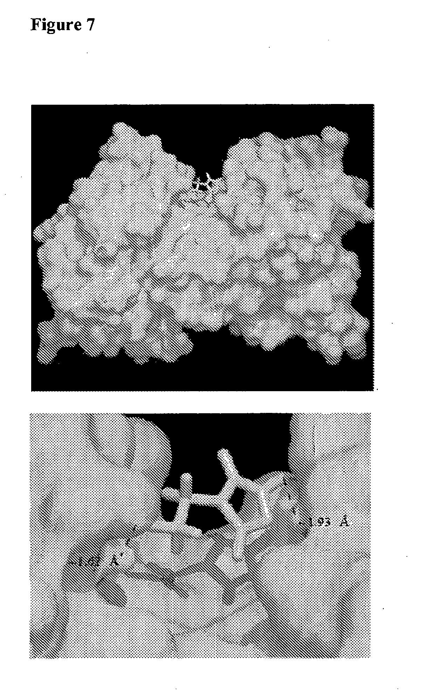

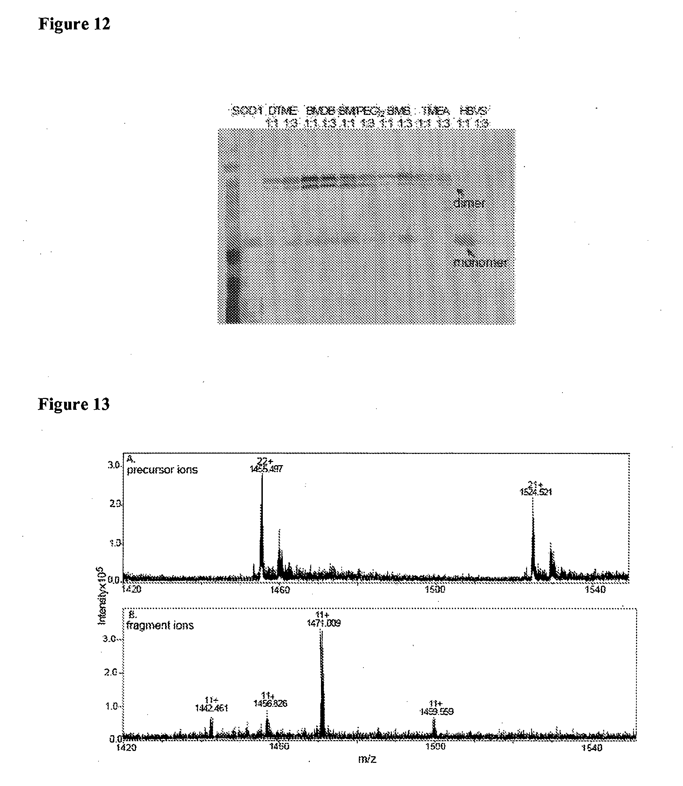

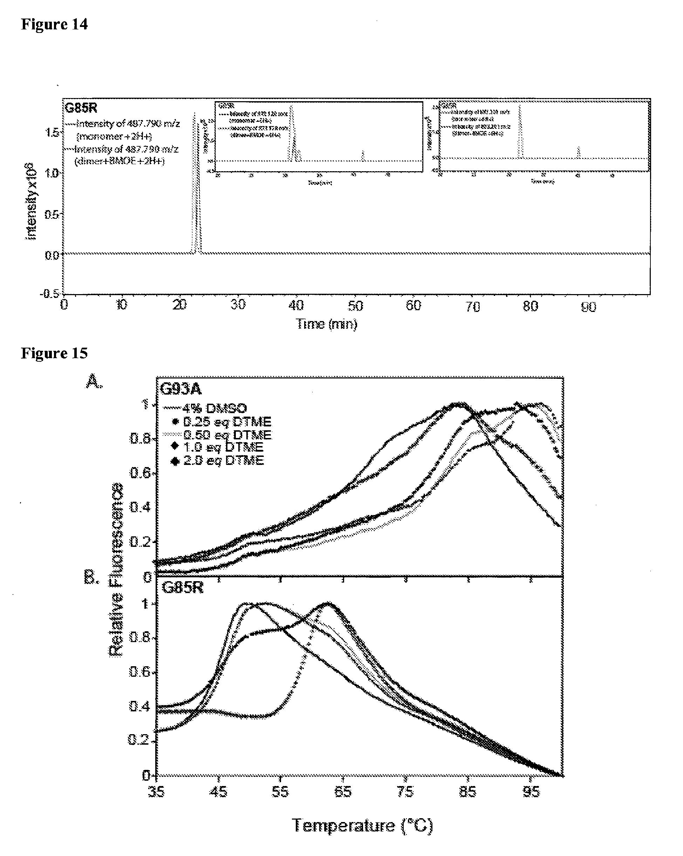

**Figure 7** shows a model of SOD1 Cysteine 111 with ½ DTME at the dimer interface. Cysteine 111 sulfur is denoted by yellow balls and ½ DTME is show in cyan. We were able to cross-link SOD1 monomers efficiently using thiol-disulfide exchange resulting in ½ DTME being bound, which is potentially amenable for peptide-based therapeutic strategies (Figure 13). Site of cross-linking confirmed via LC-FTMS analysis (Figure 14).

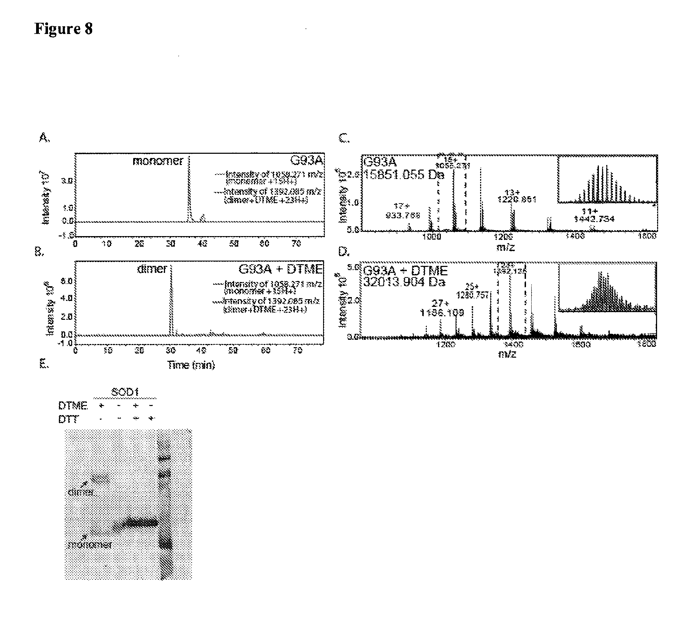

**Figure 8** shows cross-linking of SOD1 resulting in a cross-linked dimer with one cross-linker per dimer. (A) Extracted ion chromatogram for the 15 H+ charge state of the SOD1 monomer (line with peak > 3.0 at t ∼ 36 minutes) and (B) 23 H+ charge state of the DTME-cross-linked dimer (line with peak > 6.0 at t ∼ 30 minutes). (C) Mass spectrum of non-cross-linked G93A SOD1 and (D) DTME cross-linked G93A SOD1.
   On Figure 8A, the curve with peak > 3.0 at t=37 minutes is Intensity of 1058.271 m/z (monomer + 15 H+). On Figure 8B, the curve with peak > 6.0 at t=30 minutes is Intensity of 1392.085 m/z (dimer + DTME + 23 H+)
   In addition to cross-linking WT SOD1, G93A was cross-linked using DTME and BMOE at a 1:1 molar concentration. The spectra observed are consistent with the molecular weight of G93A plus one cross-linker, BMOE or DTME. (E) Chemical cross-linking of WT SOD1 with a reductively labile molecule. One of the maleimide cross-linkers tested, DTME, has a disulfide bond in its spacer arm which can be cleaved by reducing agents such as DTT. WT SOD1 was cross-linked with a 1:1 molar ratio of DTME and analyzed by SDS PAGE gel in both the presence and in the absence of reducing agent, DTT. The SOD 1 cross-linked dimer became a monomer in the presence of reducing agent (Figure 8B, lane 2) suggesting that the dimer being formed is specific to the cross-linkers being used, and was not the result of cross-linker catalyzed dimer formation.

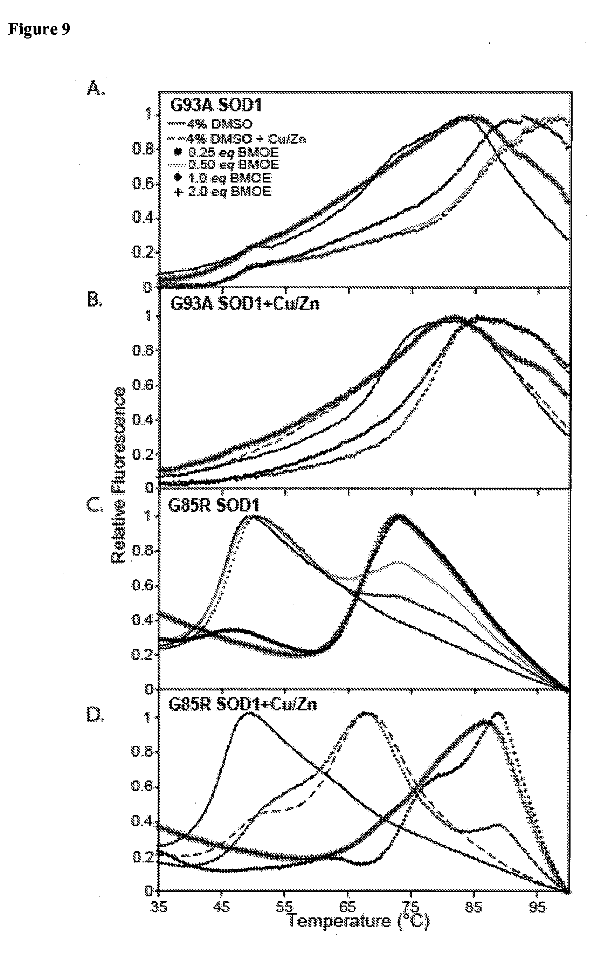

**Figure 9** depicts the stabilization of fALS-associated SOD1 variants by chemical cross-linking. See also Figure 15 (stability of mutant SOD1 measured by thermofluorescence assay). 10 µM G93A (A) and G85R SOD1 (C) were incubated with concentrations of BMOE ranging from 2.5-20 µM. 10 µM G93A (B) and G85R (D) were incubated with 20 µM copper/zinc and concentrations of BMOE ranging from 2.5-20 µM. The cross-linkers used here were resuspended in DMSO, therefore SOD1 in 4% DMSO controls were used. WtSOD1 was also investigated, however, due to its near-boiling point melting temperature the assay used here is not capable of detecting whether or not stabilization occurred (data not shown).
   G85R and G93A were stabilized in a cross-linker concentration-dependent manner by approximately 40°C and 20°C, respectively. The addition of copper and zinc had little effect on the wild-type like G93A mutant, however the addition of copper and zinc to G85R, a metal-deficient mutant, along with cross-linkers stabilizes the protein to almost wild-type levels. Figure 10 illustrates that this highly stable form of G85R has regained wild-type like superoxide dismutase activity. The above graphs represent the average of three replicates of each respective concentration; repeated in triplicate.

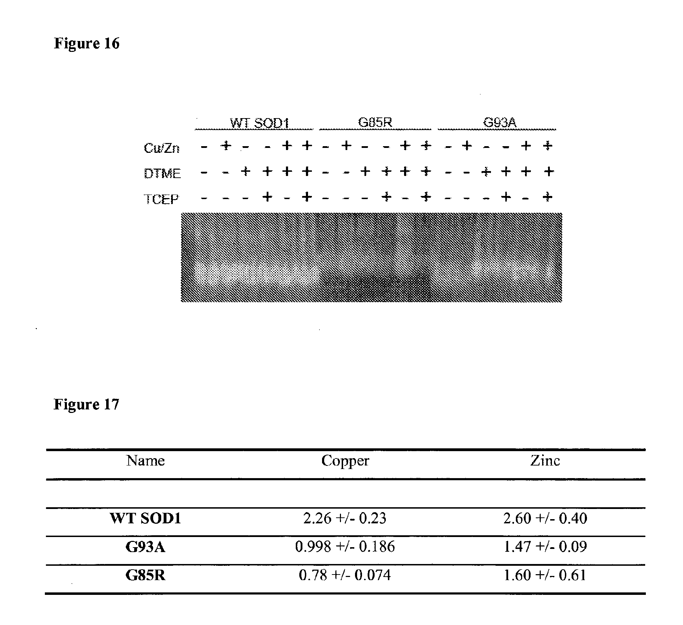

**Figure 10** depicts the rescue of fALS-variant G85R SOD1 activity by chemical cross-linking. WtSOD1 activity appears unchanged by the addition of copper, zinc, and/or BMOE. G93A, a wt-like mutant, appears to also be unaffected by the addition of copper, zinc, and/or BMOE, however it does appear to have a reduced activity compared to wild-type. G85R is a metal-deficient mutant that is inactive. In the presence of excess copper, zinc or BMOE G85R activity increases. Most notably, G85R in the presence of copper, zinc, and DTME has an even larger increase in activity. Thus, the increase in stability of G85R also corresponds to an increase in activity (Figure 16).

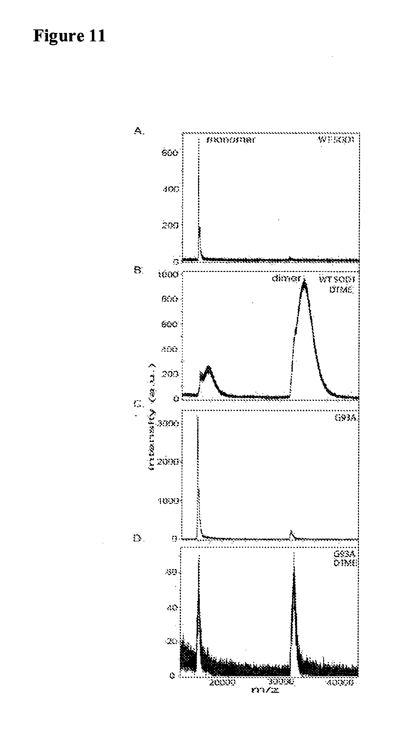

**Figure 11** depicts the presence of dimer formation in cross-linked WT SOD1 and G93A SOD1. (A.) Spectra of native (not cross-linked) wtSOD1. (B.) Spectra of DTME cross-linked wtSOD1 spectra. The majority of the species observed is dimeric wtSOD1. (C.) Spectra of native G93A. (D.) Spectra of DTME cross-linked G93A. In addition to cross-linking WT SOD 1, G93A was cross-linked using DTME and BMOE at a 1:1 molar concentration. The second peak of lower molecular weight could correspond to either monomeric SOD1 or the [M+2H]²⁺ dimer.
**Figure** 12 shows by Western blot analysis how cysteine cross-linking results in SOD1 dimer formation. Based on the crystallographic distances between the two cysteine residue rotomers, we used maleimide cross-linkers with spacers ranging from 8.0 to 14.7 Å and one vinylsulfone cross-linker with a spacer of 14.7 Å. Prior to cross-linking SOD1 was DTT treated to remove adventitious sulfane sulfur (de Beus, 2004 #1268). DTT-reduction was quenched by C18 reverse chromatography, elution of SOD1 into 50/50 H20/ACN, 0.1% formic acid, and buffer exchange of this eluent into PBS, pH 7.4 using an amicon YM10 concentrator. Cross-linking was performed at 1:1 or 1:3 molar ratio of SOD 1 to cross-linker for 1 hour at room temperature and analyzed by western blot using a polyclonal antibody to SOD1 (SOD100, Stressgen) (A) 1:1 and 1:3 chemical cross-linking of WT SOD1 with various maleimides and a vinylsulfone.
**Figure 13** shows fragmentation suggesting thiol-disulfide exchange of DTME. (A) The G93A dimer (precursor ion) at 1455.4 modified by one cross-linker. (B) Funnel skimmer dissociation of the G93A cross-linked dimer (fragment ions). The m/z of 1442.4 corresponds to the G93A monomer, m/z of 1456.8 corresponds to the G93A monomer modified by the cleaved cross-linker, m/z of 1471.0 corresponds to the G93A monomer modified by one cross-linker, and m/z of 1499.7 corresponds to G93A modified by two cross-linkers.
**Figure 14** shows Cys111 is a representative site of chemical cross-linking. In order to identify the representative site of cross-linking, G85R was cross-linked with BMOE and digested with Glu-C. The peptides were then analyzed using Fourier transform mass spectrometry (LC-FTMS). The peptide data generated from the LC-FTMS runs were submitted for MASCOT searching and the results for the non-cross-linked and cross-linked samples were compared. Two Mᵣ's that were in the cross-linked but not in the native sample were 5232.674 (m/z 873.120) and 5347.700 (m/z 892.291). Extracted ion chromatograms (EIC) were created for each of the above m/zs using a 0.01 Da error tolerance: line with peak > 1.5 at t ∼ 22.5 minutes (cross-linked) and line with peak ∼ 1.5 at t ∼ 23 minutes (non-cross-linked). The m/z 487.790 (Mᵣ 973.563; residues 1-9, acetylated N-terminus) is observed in both samples and presented as a positive control. Following comparison of the EICs, 5232.674, 5347.740, and other peptide candidates for being cross-linked where inputed into the MS-Bridge website, which compares the Mᵣs to a list of all possible cross-linked peptide Mᵣs plus the molecular weight of the cross-linker (BMOE 220.05 Da). MS-Bridge identified both 5232.674 and 5347.740 as being involved in a cross-link (residues 103-125 to residues 103-126) and (residues 103-126 to residues 103-126) respectively. The predicted cross-link for 5232.674 had a 6.19 ppm accuracy whereas 5347.700 had a 5.88 ppm accuracy. Therefore, since only one Cysteine falls within these predicted cross-links, LC-FTMS/MS data revealed the site of cross-linking as Cys111. Notably, due to the complexity of cross-linking no MS/MS data was obtained for the predicted cross-linked peptides.
**Figure 15** shows stabilization of fALS-associated SOD1 variants by chemical cross-linking. (A.) 10 µM G93A incubated with 2.5-20 µM DTME. (B.) 10 µM G85R incubated with 0-20 µM DTME. Similar results to BMOE (Figure 9) were observed for DTME.
**Figure 16** shows rescue of fALS-variant G85R SOD1 activity by chemical cross-linking. SOD1 activity was monitored using a nitro blue tetrazoleum (NBT) 12.5% native polyacrylamide gel-based assay (69-72). WT or fALS-associated SOD1 variants were incubated in the presence or absence of the following: 2-fold excess copper and zinc; equimolar DTME; and TCEP, which cleaves any DTME mediated cross-link. G85R is a metal-deficient mutant that is essentially inactive, whereas wt SOD1 and G93A SOD1 are fully active. In the presence of excess copper, zinc or DTME, however G85R activity increases. Notably, G85R in the presence of copper, zinc, and DTME had the largest increase in activity, and cleavage of the DTME-mediated cross-link by TCEP resulted in a loss of activity. Similar results were obtained for BMOE.
**Figure 17** shows metal content per monomer for wild-type SOD1 and selected mutants.

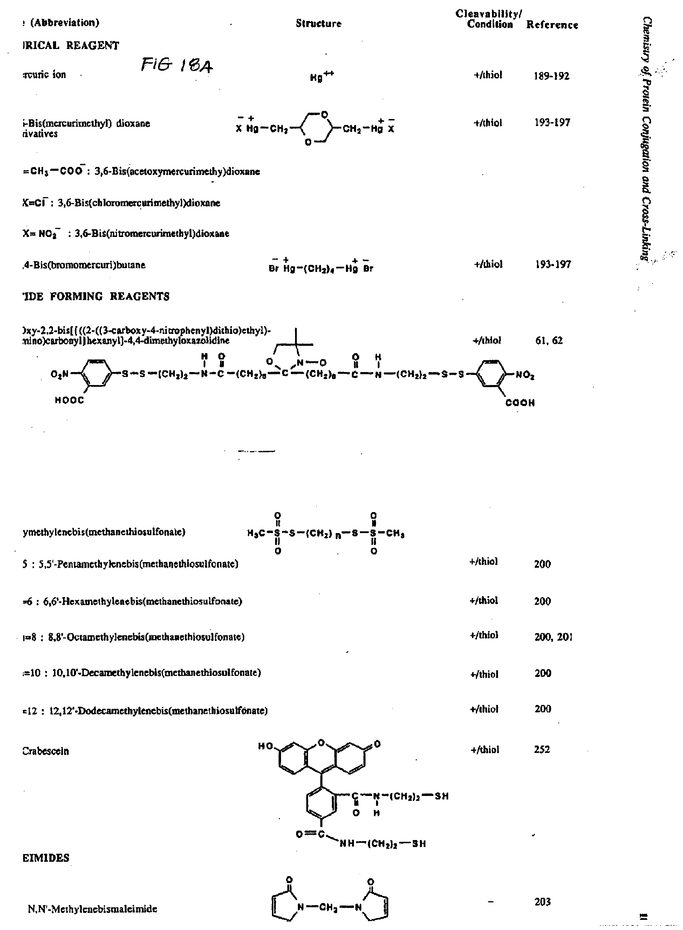

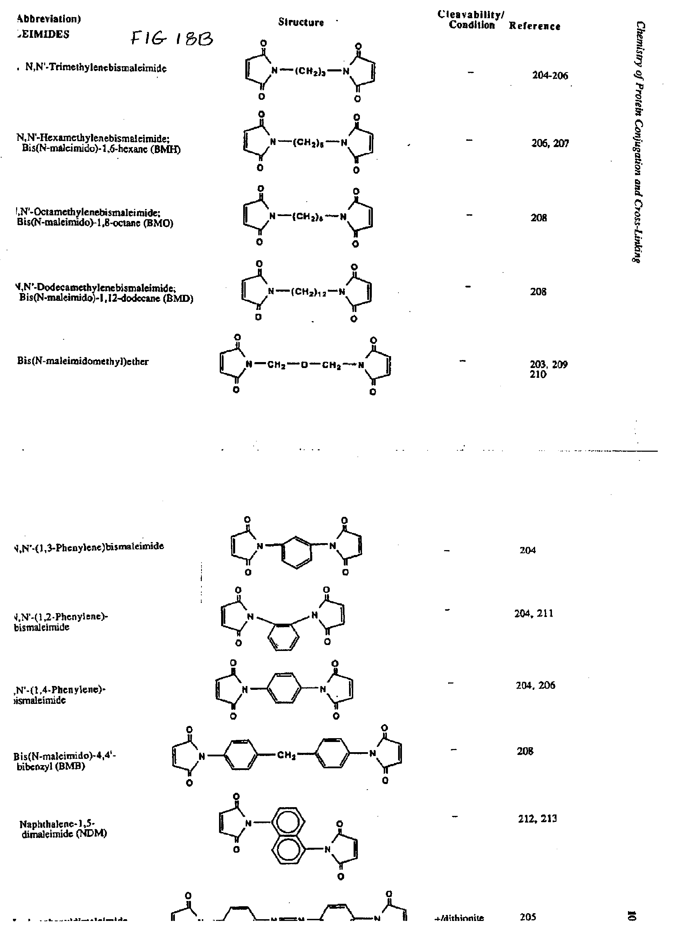

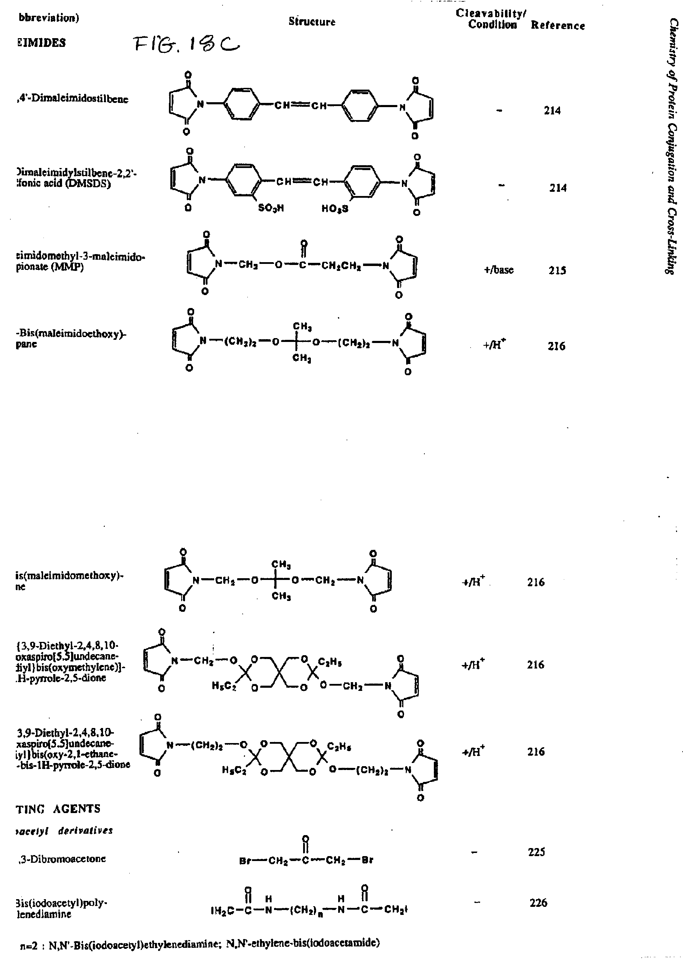

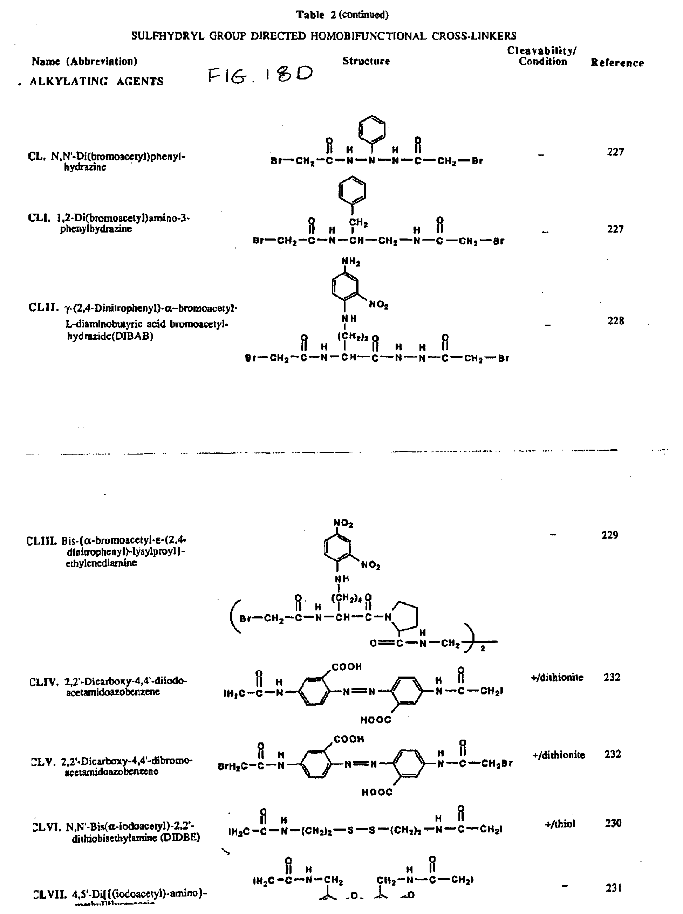

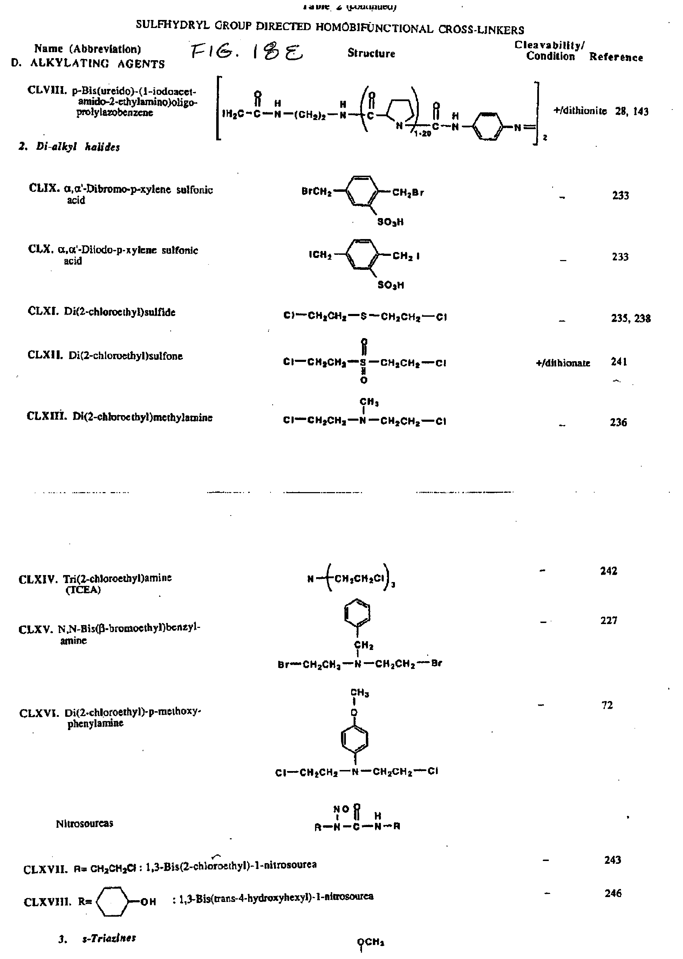

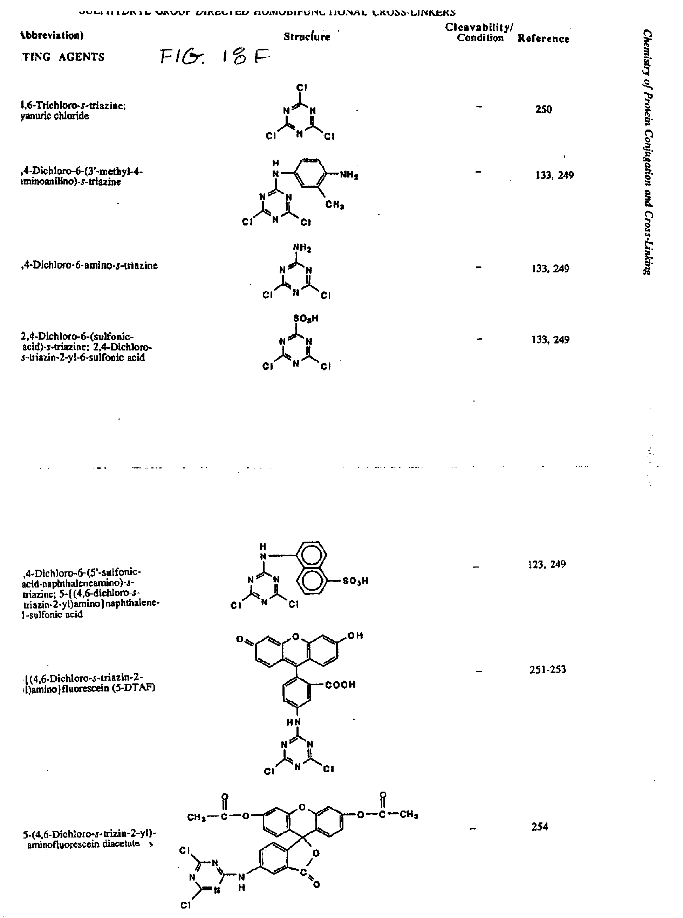

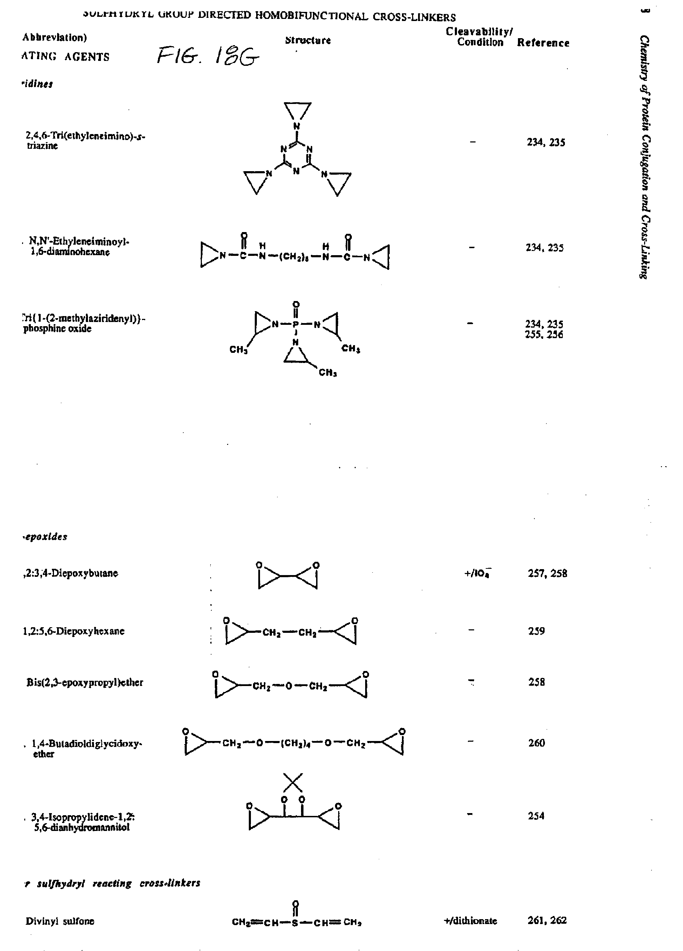

**Figures 18A-G** shows various types of cross-linkers useful in the invention.

### DETAILED DESCRIPTION OF THE INVENTION

We developed strategies for increasing the thermostability of fALS-associated SOD1 variants that took advantage of the unique nucleophilicity of cysteine residues in general, and the proximity of the Cys111 of adjacent SOD1 monomers in particular. We present both maleimide and thiol-disulfide exchange-mediated stabilization of SOD1 using two adjacent cysteines (Cys111) on each respective SOD1 monomer. Mass spectrometry data is consistent with one equivalent of cross-linker producing one equivalent of dimer, and a reductively labile cross-linker (DTME), ruled out the occurrence of cross-linker-catalyzed reactions. Reduction of DTME via TCEP also ruled out Cys111-mediated effects that do not involve intersubunit disulifides (Figure 16).

Chemical cross-linking stabilized G93A ∼20°C and excess copper, zinc, and chemical cross-linking stabilized G85R ∼45°C, which is the highest ever achieved for SOD1, and as far as we know, for any disease-associated protein. G85R is among the fALS variants that are found as monomers in vivo, suggesting that our approach may be effective for similar variants. In addition to stabilizing G85R, excess copper, zinc, and chemical cross-linking increased its enzymatic activity. It is expected that some if not all of the prevalent SOD1 variants, including the five most prevalent fALS SOD1 variants (D90A, A4V, E100G, H46R, and I113T) may also show increased stabilization and enzymatic activity.

Notably, the increased activity achieved here by targeting residues distal to the active site is in contrast to the most popular strategy for designing pharmacological chaperones, which involves binding reversible inhibitors to the active site. For such active site inhibitors a primary source of toxicity, or lack of efficacy against some mutants, is the enzyme inhibition that is fundamental to the approach, and that results in compromising on a dose that may be suboptimal for stabilization. In contrast, our approach, interacting at the dimer interface, both stabilizes SOD1 and increases SOD1 activity for at least some inactive mutants. The use of certain representative cross-linking agents described here are also suited for use as scaffolds for the design of high affinity and/or specificity compounds that may serve as potential therapeutics that can be validated in ALS cell culture and mouse models.

In order to stabilize the human SOD1 dimer while minimizing the potential for toxicity (off target binding to other proteins), we took advantage of the presence of two symmetrically arranged Cys 111 residues on opposite sides of the dimer interface that are separated by ∼9 or 13 angstroms, depending upon Cys rotomer (Figure 7); the high nucleophilicity (unique reactivity) of cysteine residues in general; and the entropic benefits of cross-linking. Compound binding strength was increased through the use of covalent bonding, such that binding is essentially irreversible *in vitro.*

Other cysteine-directed classes of compounds include mercurial reagents, peptides, non-peptide disulfide forming reagents, bismaleimides, and alkylating agents (e.g., iodoacetamide). For comparing these different classes of cross-linkers, we considered general solubility (the log of the octanol-water partition coefficient), expressed as logP and toxicity, expressed as IC₅₀ or LD₅₀, the lethal dose for 50% of exposed cells or animals, respectively. Lipinski's rules showed that compounds with logP values of no greater than 5 (ideally between 1-3) are considered "drug-like", and compounds with logP less 1 can be sufficiently dissolved in aqueous solutions. A representative number of compounds (from 3-350 depending upon availability) in each of the classes can be searched in literature by PubChem. The classes rank in following order of solubility in water, *from highest to lowest*: peptides (highly variable and sequence dependent, with logP ranges from -3 to 12 for 350 peptides) > bismaleimides (XlogP ∼1) > alkylating agents (XlogP ∼2.5) > non-peptide disulfide forming reagents (XlogP∼4), mercurial (not searched due to high toxicity, see below). The rank for toxicity from *lowest to highest* is as follows: peptides (variable but often tolerated at biological concentrations of >10 mM), bismaleimides (note: the most commonly studied maleimide, *N*-ethylmaleimide is hepatotoxic at 0.075 mg kg⁻¹ in rats, and a second maleimide had 2 µm IC₅₀ in cell culture and <40mg kg⁻¹ in mice) < alkylating agents [1,3-dibromoacetone (3 g kg⁻¹ in mice), bis-epoxides (range of 4-20 g kg⁻¹), and some vinyl sulfones (1 g kg⁻¹)] while other alkylating agents [phenylhydrazine (80 mg kg⁻¹) and N,N'-ethylenebis-(iodoacetamide) (9 mg kg⁻¹)] are not recommended due to their LD₅₀ values in human) < mercurial derivatives (variable, but some compounds are in the range of 10-40 mg/kg for humans). Of these compounds, certain alkylating agents could be tolerated at very high doses.

### Prophetic Embodiments

### Determine if Cys111-cross-linker-mediated stabilization is applicable to the most prevalent fALS SOD1 variants and to oxidized WT SOD1

In preliminary results we used homobifunctional, Cys111-specific, maleimide cross-linkers to tether the individual SOD1 monomers of the G93A and G85R SOD1 variants. We achieved what is to our knowledge an unprecedented level of small-molecule mediated stabilization for any protein by increasing G85R SOD1's melting point by ∼45 °C, while also restoring nearly full activity to the otherwise inactive G85R variant. To determine this broader therapeutic potential, the five most prevalent fALS SOD1 variants (D90A, A4V, E100G, H46R, and I113T), C6G, and oxidized WT SOD1 will be analyzed in the following way:
A. Express and purify the prevalent SOD1 D90A, A4V, E100G, H46R, I113T, and C6G variants.
B. Determine if Cys111 homobifunctional maleimides: 1) tether the dimers of these variants at Cys111 using mass spectrometry; 2) stabilize fALS SOD1 variants using a "thermofluorescence" stability assay; and 3) improve enzymatic activity. Determine the sites of compound binding using mass spectrometry, and use the compound bound to SOD1 Cys6 as a proxy for side reaction with other free cysteines.
C. Determine if homobifunctional maleimides slow the rate of oxidation and monomerization caused by SOD1's enzymatic reaction product, peroxide, and also determine if homobifunctional maleimides stabilize WT SOD1 that was previously oxidized.

### Developing peptide-based Cys111 cross-linking and SOD1 stabilizing molecules

Using both rational, predefined constraints and iterative, experimentally determined constraints, a combinatorial library of ∼2000 peptides that are amenable to thiol-disulfide exchange-mediated SOD1 dimer stabilization may be parsed from the >20 million possible di-, tri-, tetra- and cyclic peptides and used. These peptides may be synthesized by a high-throughput service facility; multiplexed mass spectrometry assays determine the on-rate of compounds and their off-rate in the presence of the physiological reducing agent, glutathione; multiplexed stability assays are performed, and dismutase activity is determined for the best 200 compounds.

To test different spacer arm lengths and different chemical space, we will create R-Cys-Cys-R, R-Cys-X-Cys-R₁, and R-Cys-X-X-Cys-R cross-linkers where R is a variable group attached by a disulfide bond to the peptide's cysteinyl sulfur, and X is a variable amino acid that can be comprised of the 20 naturally occurring amino acids and 8 unnatural amino acids. We will test the ability of these compounds to stabilize SOD1 using mass spectrometry, thermofluorescence, and activity.

We will create cyclic peptides by coupling the most active peptides from Aims 2A-C via their Cys-Cys disulfide bonds. Testing of these compounds will be as above.

We will generate quantitative structure activity relationship models using the experimental results and use these to create an additional 200 optimized peptides, which are tested as above and are expected to have improved performance.

We expect to develop and characterize the first generation of peptide-based, SOD1 Cys111 cross-linkers. The relatively low cost of peptide synthesis allows a concomitant increase in the scale of the in vitro structure activity relationships. One goal is that structure-activity relationships will arise and encourage qualified groups, collaborators, or companies to undertake the design of peptidomimetics. Structure-activity relationships are a prerequisite for creating peptidomimetics, which involves the further "analoging" of critical residues. Another goal is to enable our own cell culture-based toxicology and bioactivity assays, which provide additional, and critical, structure-activity information. In other studies, we routinely immunopurify SOD1 from cell culture and animals, and the same protocol could be used in future cell culture or animal studies as a bioassay for cross-linked SOD1.

We propose to first discover peptides that can bind SOD1 and then to create cyclic analogues of these peptide by binding them through their N-and C-terminal cysteine residues to other active peptides. By using peptides we will able to explore a large amount of chemical space while subcontracting the synthesis, and concentrate on the relatively high-throughput screening. Peptides represent one relatively inexpensive way of exploring chemical space, and the potential weakness of having poor pharmacological properties that can probably only be overcome with expertise in peptidomimetics. In other words, peptides can be used to inform the synthesis of non-peptide small molecules. Peptide drugs have a low cost, robust synthesis, increased specificity in locating amino acids of interest inherently low toxicity, and at the sizes of peptides used here, virtually no immunogenicity. One major difference, and potential difference of our approach is that nominally only two amino acids, Cys-Cys are required for cross-linking SOD1, making our peptide potentially much smaller than other bioactive peptides. This will certainly help in crossing the blood brain barrier and will probably decrease digestion by peptidases.

A potential representative peptide-based approach to SOD1 stabilization is the creation of cyclic peptides. The general approach of cyclizing the active residues of a peptide using cysteine-disulfides was successful in the case of α-MSH analogues, which flanked the His-Phe-Arg-Trp essential active core of α-MSH with cysteine residues, and then cyclized using the same cysteines. Cyclic peptides have been used for almost thirty years to enhance the bioactivity and pharmacological properties of linear peptides. Another testament to their efficacy is that numerous natural product-derived antibiotics, including well known antibiotics like bacitracin, cyclosporine, and nisin (a common food preservative), are cyclic peptides. Cyclic peptide can be extraordinary stable, even to the extent that they survive the human digestive track. The resulting cyclic peptide had 60 times the biological activity. This biological half life of peptide was improved upon even farther by shortening the sequence to only seven amino acids and incorporating non-natural amino acids. There are numerous advantages to cyclic peptides over their linear analogues, including superior pharmacokinetics of both metabolism and secretion and more resistance to proteolytic degradation. Cyclic peptides are more constrained and populate a more defined molecular structure, which in turn allows better estimation of the appropriate pharmacophore when constructing peptidomimetics. We performed qualitative, 20 picosecond MM2-based molecular dynamics simulations and the difference between cysteinyl sulfur mobility in linear peptides, which could be greater than 8Ǻ, and cyclic peptides, which was on the order of 2Ǻ, was remarkable.

### Representative Analogues of the Invention

One aspect of the invention is a stabilized superoxide dismutase analogue, wherein said analogue has a tertiary structure and comprises a first SOD1 monomer, a second SOD1 monomer, and a crosslinker; said crosslinker comprises a spacer arm with length from 3 Å to 15 Å; wherein said first SOD1 monomer comprises a first cysteine at a first position corresponding to position 111 of wild-type human SOD1, and said second SOD1 monomer comprises a second cysteine at a second position corresponding to position 111 of wild-type human SOD1; and the first cysteine is covalently connected to the second cysteine by the crosslinker.

In certain embodiments, the present invention relates to any one of the aforementioned analogues, wherein the tertiary structure is substantially the same as the wild-type superoxide dismutase enzyme.

In certain embodiments, the present invention relates to any one of the aforementioned analogues, wherein the first cysteine and the second cysteine occupy the same numbered position.

In certain embodiments, the present invention relates to any one of the aforementioned analogues, wherein the first cysteine and the second cysteine occupy different numbered positions.

In certain embodiments, the present invention relates to any one of the aforementioned analogues, wherein the first cysteine is at position 111.

In certain embodiments, the present invention relates to any one of the aforementioned analogues, wherein the second cysteine is at position 111.

In certain embodiments, the present invention relates to any one of the aforementioned analogues, wherein the sequence identity of said first SOD1 monomer and said second SOD1 monomer is greater than or equal to about 85%.

In certain embodiments, the present invention relates to any one of the aforementioned analogues, wherein said first SOD1 monomer and said second SOD1 monomer have the same amino acid sequence.

In certain embodiments, the present invention relates to any one of the aforementioned analogues, wherein the length of the connection is from about 5 Angstroms to about 15 Angstroms.

In certain embodiments, the present invention relates to any one of the aforementioned analogues, wherein the length of the connection is from about 8 Angstroms to 14 Angstroms.

In certain embodiments, the present invention relates to any one of the aforementioned analogues, wherein the length of the connection is from about 9 Angstroms to 11 Angstroms.

In certain embodiments, the present invention relates to any one of the aforementioned analogues, wherein the length of the connection is about 9 Angstroms or about 13 Angstroms.

In certain embodiments, the present invention relates to any one of the aforementioned analogues, wherein the first SOD1 monomer of said analogue is the wild-type sequence or comprises a mutation selected from the group consisting of G93A, G85R, D90A, A4V, E100G, H46R, C6G, and I113T.

In certain embodiments, the present invention relates to any one of the aforementioned analogues, wherein the second SOD1 monomer of said analogue is the wild-type sequence or comprises a mutation selected from the group consisting of G93A, G85R, D90A, A4V, E100G, H46R, C6G, and I113T.

In certain embodiments, the present invention relates to any one of the aforementioned analogues, wherein said analogue retains at least 90% activity of the wild-type superoxide dismutase enzyme up to a temperature of 75 degrees C.

In certain embodiments, the present invention relates to any one of the aforementioned analogues, wherein the unfolding temperature of said analogue is increased by from 10 degrees C to 60 degrees C as compared to its uncrosslinked dimer.

In certain embodiments, the present invention relates to any one of the aforementioned analogues, wherein the unfolding temperature of said analogue is increased by from 20 degrees C to 40 degrees C as compared to its uncrosslinked dimer.

In certain embodiments, the present invention relates to any one of the aforementioned analogues, wherein the unfolding temperature of said analogue is increased by from 15 degrees C to 25 degrees C as compared to its uncrosslinked dimer.

In certain embodiments, the present invention relates to any one of the aforementioned analogues, wherein the unfolding temperature of said analogue is increased by from 30 degrees C to 50 degrees C as compared to its uncrosslinked dimer.

In certain embodiments, the present invention relates to any one of the aforementioned analogues, wherein the unfolding temperature of said analogue is increased by 20 degrees C as compared to its uncrosslinked dimer.

In certain embodiments, the present invention relates to any one of the aforementioned analogues, wherein the unfolding temperature of said analogue is increased by 40 degrees C as compared to its uncrosslinked dimer.

In certain embodiments, the present invention relates to any one of the aforementioned analogues, wherein the cross-linker is selected from the group consisting of tris[2-maleimidoethyl]amine (TMEA), Dithio-bismaleimidoethane (DTME), 1,4-bismaleimidyl-2,3-dihydroxybutane (BMDB), (1,8-bis-maleimido-diethyleneglycol (BM(PEG)2), 1,4-bis(maleimido)butane (BMB), and bis(Maleimido)Ethane (BMOE).

In certain embodiments, the present invention relates to any one of the aforementioned analogues, wherein the crosslinker is selected from the group consisting of organomercurials, maleimides, vinyl sulfones, and alkylating agents.

In certain embodiments, the present invention relates to any one of the aforementioned analogues, wherein the crosslinker comprises a spacer arm with length from about 7 Angstroms to about 11 Angstroms.

In certain embodiments, the present invention relates to any one of the aforementioned analogues, wherein the crosslinker comprises a spacer arm with length of about 9 Angstroms.

Another aspect of the invention is a pharmaceutical composition, comprising a stabilized SOD1 analogue; and a pharmaceutically acceptable carrier.

### Representative Methods of the Invention

Another aspect of the invention is a method of producing a stabilized superoxide dismutase (SOD1) analogue, comprising the step of reacting a first SOD1 monomer, a second SOD1 monomer, and a cross-linker, thereby forming said analogue.

Another aspect of the invention is a method of producing a stabilized superoxide dismutase (SOD1) analogue, comprising the step of connecting a first cysteine of a first SOD1 monomer with a second cysteine of a second SOD1 monomer.

In certain embodiments, the present invention relates to any one of the aforementioned methods, further comprising the step of replacing a naturally occurring amino acid of the first SOD1 monomer with the first cysteine.

In certain embodiments, the present invention relates to any one of the aforementioned methods, further comprising the step replacing a naturally occurring amino acid of the second SOD1 monomer with the second cysteine.

Another aspect of the invention is a stabilized SOD1 analogue of the present invention for use in treatment of a neurodegenerative disease or in prophylaxis for neurodegenerative disease, wherein the neurodegenerative disease is amyotrophic lateral sclerosis.

### Definitions

The term "analogue" refers to a molecule substantially similar in function to SOD1 protein or a fragment thereof.

The terms "percent (%) amino acid sequence identity" or "percent amino acid sequence homology" or "percent (%) identical" as used herein with respect to a reference polypeptide is defined as the percentage of amino acid residues in a candidate polypeptide sequence that are identical with the amino acid residues in the reference polypeptide sequence after aligning the sequences and introducing gaps, if necessary, to achieve the maximum percent sequence identity, without considering any conservative substitutions as part of the sequence identity. Alignment for the purpose of determining percent amino acid sequence identity can be achieved by various techniques known in the art, for instance, using publicly available computer software such as ALIGN or Megalign (DNASTAR). Those skilled in the art can determine appropriate parameters for measuring alignment, including any algorithms needed to achieve maximal alignment over the full length of the peptide sequence being used in the comparison. For example, in the context of the present invention, an analogue of SOD1 is said to share "substantial homology" with SOD1 if the amino acid sequence of said analogue is at least about 85%, at least about 90%, at least about 95%, or at least about 99% identical to wild-type.

The phrase "pharmaceutically acceptable" is employed herein to refer to those ligands, materials, compositions, and/or dosage forms which are, within the scope of sound medical judgment, suitable for use in contact with the tissues of human beings and animals, substantially non-pyrogenic, without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject chemical from one organ or portion of the body, to another organ or portion of the body. Each carrier must be "acceptable" in the sense of being compatible with the other ingredients of the formulation, not injurious to the patient, and substantially non-pyrogenic. Some examples of materials which can serve as pharmaceutically acceptable carriers include: (1) sugars, such as lactose, glucose, and sucrose; (2) starches, such as corn starch and potato starch; (3) cellulose, and its derivatives, such as sodium carboxymethyl cellulose, ethyl cellulose, and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) glycols, such as propylene glycol; (11) polyols, such as glycerin, sorbitol, mannitol, and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations. In certain embodiments, pharmaceutical compositions of the present invention are non-pyrogenic, i.e., do not induce significant temperature elevations when administered to a patient.

The term "preventing" is art-recognized, and when used in relation to a condition, such as a local recurrence (e.g., pain), a disease such as cancer, a syndrome complex such as heart failure or any other medical condition, is well understood in the art, and includes administration of a composition which reduces the frequency of, or delays the onset of, symptoms of a medical condition in a subject relative to a subject which does not receive the composition. Thus, prevention of cancer includes, for example, reducing the number of detectable cancerous growths in a population of patients receiving a prophylactic treatment relative to an untreated control population, and/or delaying the appearance of detectable cancerous growths in a treated population versus an untreated control population, e.g., by a statistically and/or clinically significant amount. Prevention of an infection includes, for example, reducing the number of diagnoses of the infection in a treated population versus an untreated control population, and/or delaying the onset of symptoms of the infection in a treated population versus an untreated control population. Prevention of pain includes, for example, reducing the magnitude of, or alternatively delaying, pain sensations experienced by subjects in a treated population versus an untreated control population.

A "therapeutically effective amount" of a compound, e.g., such as a polypeptide or peptide analogue of the present invention, with respect to use in treatment, refers to an amount of the polypeptide or peptide in a preparation which, when administered as part of a desired dosage regimen (to a mammal, preferably a human) alleviates a symptom, ameliorates a condition, or slows the onset of disease conditions according to clinically acceptable standards for the disorder or condition to be treated or the cosmetic purpose, e.g., at a reasonable benefit/risk ratio applicable to any medical treatment.

The terms "prophylactic" or "therapeutic" treatment are art-recognized and includes administration to the host of one or more of the subject compositions. If it is administered prior to clinical manifestation of the unwanted condition (e.g., disease or other unwanted state of the host animal) then the treatment is prophylactic, (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

### EXEMPLIFICATION

The invention now being generally described, it will be more readily understood by reference to the following examples which are included merely for purposes of illustration of certain aspects and embodiments of the present invention, and are not intended to limit the invention.

### Protein Expression and Purification

WtSOD1 was purchased from Sigma-Aldrich (St. Louis, Missouri). Constructs for expression of G93A and G85R in S. cerevisiae have been obtained through the generous gift of Dr. P. John Hart, Ph.D. (University of Texas Health Science Center, San Antonio). The expression and purification of G93A and G85R was carried out as previously published (26, 67). Briefly, each construct in the yeast expression vector YEp-351 was transformed into EGy118ΔSOD1 yeast and grown at 30 °C for 36-48 hours. Cultures were pelleted, lysed using 0.5 mm glass beads and a blender, and subjected to a 60 % ammonium sulfate cut. After ammonium sulfate precipitation the sample was pelleted and the supernatant was diluted with 0.19 volumes to a final concentration of 2.0 M ammonium sulfate. This sample was then purified using a phenyl-sepherose 6 fast flow (high sub) hydrophobic interaction chromatography column (GE Life Sciences) using a 300 mL linearly decreasing salt gradient from a high salt buffer (2.0 M ammonium sulfate, 50 mM sodium phosphate, 150 mM sodium chloride, 0.1 M EDTA, 0.25 mM DTT, pH 7.0) to a low salt buffer (50 mM sodium phosphate, 150 mM sodium chloride, 0.1 M EDTA, 0.25 mM DTT, pH 7.0). Samples containing SOD1 were eluted between 1.6-1.1 M ammonium sulfate and identified using SDS PAGE analysis, pooled and exchanged to a 10 mM Tris, pH 8.0 buffer. The protein was then loaded onto a Mono Q 10/100 anion exchange chromatography column (GE Life Sciences) and eluted using a 200 mL linearly increasing salt gradient from a low salt buffer (10 mM Tris, pH 8.0) to a high salt buffer (10 mM Tris, pH 8.0, 1 M sodium chloride). The gradient was run from 0-30% 10 mM Tris, pH 8.0, 1 M sodium chloride and SOD1 eluted between 5-12% 10 mM Tris, pH 8.0, 1 M sodium chloride. SOD1 protein was confirmed via SDS PAGE, western blot, MALDI-TOF, and FTMS.

### Cross-linking and Western Blots

Wt, G93A, or G85R SOD1 were incubated with 5-25 mM DTT for approximately 20 minutes and either buffer exchanged using Amicon Ultra-4 centrifugal spin concentrators (MWCo 10K) or using reversed phase chromatography (ZIPTIP, Millipore, Inc). Samples cleaned by ZIPTIPs were also subjected to incubation with 5 mM EDTA. SOD1 samples that were buffer exchanged using Amicon concentrators were exchanged into in HPLC water, whereas ZIPTIP samples were further exchanged after ZIPTIP into PBS, pH 7.4 or HPLC water. DTT reduced SOD1 was incubated at a 1:1 (20 µM:20 µM or 10 µM:10 µM) or 1:3 (20 µnM:60 µM or 10 µM:30 µM) ratio of protein to cross-linker.

A variety of cross-linkers (Thermo-Fisher Scientific) were used: Dithio-bismaleimidoethane (DTME, spacer arm 13.3Å), 1,4-bismaleimidyl-2,3-dihydroxybutane (BMDB, spacer arm 10.2Å), 1,8-bis-Maleimidodiethyleneglycol (BM(PEG)₂, spacer arm 14.7Å), 1,4 bismaleimidobutane (BMB, spacer arm 10.9Å), Tris[2-maleimidoethyl]-amione (TMEA, 10.3Å) and 1,6-Hexanol-bis-vinylsulfone (HBVS, spacer arm 14.7Å). Cross-linking was achieved by incubating the reaction in either PBS pH 7.4 or water at room temperature for 1 hour. After an hour the reactions were analyzed on a 15% SDS-PAGE gel with a non-cross-linked control, transferred to nitrocellulose membrane and western blotted using a polyclonal antibody to SOD1. Repeated in triplicate.

In addition, DTME is a cleavable sulfhydryl-sulfhydryl cross-linking agent. Therefore, a cross-linking reaction containing 1:1 molar ration of wtSOD1 to DTME was performed at room temperature for one hour. After cross-linking, the reaction was split in half and half of the sample was run in a sample buffer containing DTT (reducing) and the other half in one containing no DTT (non-reducing). These samples along with non-cross-linked controls were then analyzed on a 15% SDS PAGE gel and western blotted as above.

### Matrix Assisted Laser Desorption Ionization (MALDI)-time of flight (TOF)

WtSOD1 and G93A SOD1 were DTT treated and cross-linked at a 1:1 molar ratio as described above. wtSOD1 was cross-linked with all the cross-linkers mentioned previously whereas G93A was cross-linked with DTME and bis(maleimido)ethane (BMOE, spacer arm 8.0Å). BMOE was used because of its shorter spacer arm length. After cross-linking, 1 µl of sample was spotted on a MALDI target containing 1 µl of matrix, 20 mg/mL sinipic acid, and analyzed on a Bruker Daltonics Microflex. The MALDI was calibrated each time using a high molecular weight protein calibration standard, Protein Calibration Standard I (Bruker Daltonics). The MALDI-TOF was operated in linear mode using a laser power of between 72-90%. MALDI-TOF spectra were of cross-linked and non-cross-linked samples were analyzed using FlexAnalysis software (Bruker Daltonics). Repeated in triplicate.

### Liquid Chromatography (LC)-Fourier Transform Mass Spectrometer (FTMS) and Funnel Skimmer Dissociation (FSD)

G93A was cross-linked using DMTE or BMOE as previously described at a 1:1 molar ratio (5 µM:5 µM). After one hour, 3% acetonitrile and 1% formic acid was added to the sample and spun at 14,000 RPM for 10 minutes to pellet any precipitated protein. The cross-linked sample was placed in an autosampler and 1 µL of cross-linked sample was aspirated into either a Proxeon ID HPLC or Eksigent 2D UPLC with the following gradient: 3-50% B for 30 minutes, 50-95% B for 7 minutes, 95% B for 5 minutes, 95-3% B for 1 minute, and 3% B for 15 minutes. Buffer A is HPLC water with 0.1 % formic acid and buffer B is 100% acetonitrile, 0.1 % formic acid. After liquid chromatography, the sample was ionized using nanospray ionization and analyzed using a 94 Tesla Bruker Daltonics FTMS. The FTMS was controlled using Apex control software and source parameters were controlled using the Apollo II software. Spectra (monomeric and dimeric G93A) were collected using a skimmer 1 voltage of 35-40V, whereas funnel skimmer dissociation was used to fragment the cross-linked G93A by increasing skimmer one voltage 140 volts. LC-FTMS data of cross-linked and non-cross-linked G93A were analyzed using Data Analysis software (Bruker Daltonics). These experiments were repeated in triplicate.

### Peptide Sequencing using LC-FTMS (Bottom-up Analysis)

G85R was cross-linked using BMOE as previously described at a 1:1 molar ratio (5 µM:5 µM). After one hour, the cross-linked protein was heated at 99°C for 30 minutes and then incubated with 10 mM TCEP for 10 minutes. The heated and reduced cross-linked G85R was then incubated with 1.5 µL 0.5 mg/mL Glu-C at 30°C overnight. The digested sample was spun at 14,000 RPM for 10 minutes and then injected into the Eksigent UPLC using the gradient above. After liquid chromatography, the samples were introduced using nanospray ionization and MS/MS data was collected using CID. Compounds were identified using Bruker Daltonics Data Analysis software, deconvoluted, and exported to a generic mascot file. Cross-linked and non-cross-linked analysis was performed by uploading the generic mascot files into the MASCOT search engine selecting none as the enzyme, using the NCBIr database, with a 1.2 Da (MS error tolerance) and 0.6 Da (MS/MS error tolerance). MASCOT searches for the non-cross-linked and cross-linked samples were compared and m/z's identified in the cross-linked sample and not in the non-cross-linked sample were submitted to an MS Bridge (protein prospector, UCSF) search using BMOE as the cross-linker (220.05 Da). MS Bridge searches all the potential molecular weights of cross-linked peptides plus the molecular weight of the cross-linker. Peptides identified from the MS Bridge search as being involved in cross-linked were further characterized by extracted ion chromatograms in the Data Analysis software.

### Metal Analysis

Metal analysis was preformed using inductively coupled plasma mass spectrometry (ICP-MS) at the University of Georgia Chemical Analysis Lab (Athens, GA). Briefly, buffer alone was sent for analysis as a blank along with 1 µM of each variant; analysis was repeated in triplicate. In addition, 5 µM of each variant was analyzed using the FTMS in ESI mode using the direct infusion method.

### Thermofluorescence Stability Assay

The melting curve of G93A and G85R SOD1 were monitored in the presence or absence of DTME or BMOE and an excess of both copper and zinc where an increase in melting temperature suggests binding and an increase in protein stability. Therefore, protein samples in the absence of cross-linker (which are resuspended in DMSO) or copper and zinc were analyzed in the presence of 2-4% DMSO to determine the effects of DMSO on protein stability. In the first sequence of reactions, 10 µM of mutant SOD1 was incubated with increasing concentrations of 0-20 µM DTME or BMOE, incubated with 20X SYPRO™ Orange and added to a 96-well plate. Alternatively, in the second sequence of reactions, 10 µM of mutant SOD1 was incubated with 20 µM copper, 20 µM zinc, and 0-20 µM BMOE or DTME. The melting temperature of the protein was monitored using an RT-PCR machine (Applied Biosystems) with a 0.3 °C increase in temperature every minute from 25-100 °C. Data was analyzed by subtracting a dye alone blank from each respective well, normalized to 1, and temperature versus fraction of unfolded protein was graphed. Subtracting a DTME or BMOE, copper, zinc, dye blank gave similar results as subtracting a dye alone blank. Repeated in triplicate.

### SOD1 Activity Assay

SOD1 activity was monitored using a nitro blue tetrazoleum (NBT) gel based assay (69-72). 10 µg (∼42 µM) of wt or mutant SOD1 was incubated in the presence or absence of 80 µM copper and zinc and/or 42 µM BMOE, then analyzed on a 12.5% polyacrylamide gel. Alternatively, 10 µg (∼42 µM) of wt or mutant SOD1 was incubated in the presence or absence of 80 µM copper and zinc and/or 42 µM DTME. The DTME cross-linked samples were divided in half, where one half was incubated with 10 mM TCEP and one was not, then analyzed on a 12.5% polyacrylamide gel. The gel was stained using a solution containing 50mM potassium phosphate, pH 7.8, 1 tablet NBT (10 mg/tablet), and 0.1 mg/mL riboflavin for 45 minutes in the dark. After the 45 minute incubation 1 µL/mL TEMED was added and the gel was exposed to light for 2 minutes. Superoxide radicals create insoluble blue formazon from NBT. SOD1 acitivity is seen as a colorless band since SOD1 scavenges the superoxide, thus inhibiting the blue color formation. Repeated in triplicate.

### RESULTS

### Cross-linking Using Extrinsic Maleimide Functional Group Cross-Linker to Stabilize Dimeric SOD1

All of the maleimide cross-linkers tested that had chain lengths in the range of 8-14 A resulted in the stabilization of SOD1 dimer, including Dithio-bismaleimidoethane (DTME, spacer arm 13.3Å), 1,4-bismaleimidyl-2,3-dihydroxybutane (BMDB, spacer arm 10.2Å), 1,8-bis-Maleimidodiethyleneglycol (BM(PEG)₂, spacer arm 14.7Å), 1,4 bismaleimidobutane (BMB, spacer arm 10.9Å), and Tris[2-maleimidoethyl]amione (TMEA, 10.3Å) (Figure 12). Alternatively, the one vinylsulfone tested, 1,6-Hexanol-bis-vinylsulfone (HBVS, spacer arm 14.7Å), did not result in formation of dimeric SOD1 (Figure 12).

To investigate the stoichiometry of cross-linker binding, we compared the molecular weights of cross-linked (DTME or BMOE) versus non-cross-linked G93A variants using liquid chromatography (LC)-Fourier transform mass Spectrometry (FTMS). The experimental molecular weight for the monomeric, non-cross-linked, species was determined to be 15851.055 Da (theoretical 15850.889 Da), and the experimental molecular weight for the dimeric, DTME cross-linked species was determined to be 32013.904 Da (0.244 Da difference from the theoretical value of the molecular weight of two G93A monomers plus one DTME (312.37 Da)). The experimental molecular weight of dimeric DTME cross-linked G93A, 32013.904 Da, suggests that one equivalent of cross-linker produced one equivalent of dimer (Figure 8), consistent with the binding of a single crosslinker to two monomers. Similar results were obtained for G93A and BMOE cross-linking (data not shown).

To rule out the occurrence of cross-linker-catalyzed reactions, a reductively labile cross-linker, DTME, was used and cross-linking was monitored using western blotting. Cross-linking using DTME resulted in dimeric SOD1, however in the presence of reducing agent only monomer was observed (Figure 8E), suggesting that the creation of SOD1 dimer was specifically due to bound chemical cross-linkers.

### Thiol-Disulfide Exchange Mediated SOD1 Dimmer Stabilization

The mass of intact SOD1 and MS/MS fragmentation data using Funnel Skimmer Dissociation (FSD) of the DTME cross-linked G93A variant (precursor ions) revealed a unique reaction mechanism, thiol-disulfide exchange, for cross-linking SOD1 (Figure 13). First, the intact SOD1 and the SOD1 with ½ DTME eluate at different retention times in the liquid chromatography run suggesting they are unique species thus ruling out the possibility of fragmentation at the disulfide of DTME. Second, fragmentation occurs preferentially at the site of cross-linking. The species modified by one half DTME suggests that exchange occurred between the sulfhydryl-sulfhydryl bond within DTME's spacer and the thiol moiety of a cysteine residue on G93A. Finally, the possibility of thiol-disulfide exchange was further confirmed by modeling half of DTME at the dimer interface, specifically Cys111, of the SOD1 structure ((2C9V (Strange, 2006 #95))) (Figure 7). Thus, we were able to cross-link SOD1 monomers efficiently using numerous molecules (Figure 12) and two distinct reaction mechanisms, namely maleimide (Figure 8, Figures 11, and Figure 12) and thiol-disulfide exchange (Figure 13), with the latter being suitable for peptide-based therapeutic strategies.

### Cys111 is the Site of Chemical Cross-linking

In order to identify the site of cross-linking, we compared proteolysis and LC-FTMS/MS data, which provided sequence data of the digested peptides, for non-cross-linked and cross-linked samples (Figure 14). Due to the protease resistance of WT and G93A SOD1, likely conferred via metal binding and an intra-molecular disulfide bond, the G85R variant, which is less protease resistant, was used. The m/z 487.790 (Mᵣ 973.563; residues 1-9, acetylated N-terminus) is observed in both samples and is presented as a positive control, highlighting similar intensities of the peptide in the cross-linked and non-cross-linked samples. We observed two Mᵣ's that were in the cross-linked but not in the native sample: 5232.674 (m/z 873.120) and 5347.700 (m/z 892.291). MS-Bridge identified both 5232.674 and 5347.740 as being involved in a cross-link (residues 103-125 of one monomer cross-linked via BMOE to residues 103-126 of a second monomer) and (residues 103-126 of one monomer cross-linked by BMOE to residues 103-126 of a second monomer), respectively. The predicted cross-link for 5232.674 had a 6.19 ppm accuracy whereas 5347.700 had a 5.88 ppm accuracy. Therefore, Mass spectrometry confirmed that cross-linking was stoichiometric and occurred mainly through Cys111, and that cross-reactivity with other SOD1 cysteine residues Cys6, Cys57, and Cys146, was relatively low (Figure 14). Notably, due to the complexity of cross-linking there was a lack of precursor ion fragmention of the cross-linked peptides.

### Chemical Cross-linking Stabilizes the G93A and G85R Dimers

Sypro orange binds preferentially to hydrophobic patches that become exposed as a protein unfolds. A fluorescence-based assay monitoring Sypro orange binding as a function of temperature was used to observe trends in the unfolding temperatures of both G93A and G85R. This assay is not reversible, probably as the result of aggregation of SOD 1, and while the unfolding temperatures resemble the previously observed melting temperatures of SOD1, they are not proper thermodynamic stabilities. WT SOD1 could not be monitored using this assay because its melting temperature is at the limit of detection (∼100C). We observed unprecedented increases in the stability of the two mutants analyzed (Figure 9 and Figure 15). G93A SOD1, for example, was stabilized by ∼20 °C; incubation with excess copper and zinc had no affect on G93A stability; and the lower concentrations of cross-linker had a greater effect on G93A stability. G85R SOD1, on the other hand, was stabilized ∼ 20 °C in the presence of excess copper and zinc; was further increased ∼45°C (from ∼40°C to 85°C) in the presence of copper, zinc, and cross-linker; and the higher concentrations of cross-linker had a greater effect on G85R stability. Thus, the degree of stabilization achieved here by what are essentially (covalent) pharmacological chaperones is the highest ever achieved for SOD1, and as far as we know, for any disease-associated protein.

### Activity of the fALS Variant G85R SOD1 is Restored by Stabilizing the SOD1 Dimer

In addition to stability we investigated the affect chemical cross-linking had on SOD1 activity using a gel-based assay. WT and G93A SOD1 activity were unaffected by the addition of copper, zinc, or chemical cross-linking. However, in addition to increasing the thermostability of G85R SOD1, chemical cross-linking increased its qualitative metal binding affinity, resulting in the transformation of G85R SOD1 from a protein that is catalytically inactive in vitro and in vivo, to a protein that is active in vitro and potentially in vivo (Figure 10). The increase in G85R activity observed during DTME cross-linking was reversed upon cleavage of DTME's internal disulfide bond using TCEP (Figure 16). Thus, in addition to the improvement in the thermostability of G85R SOD1, these compounds have the potential to increase the resistance of the organism to oxidative stress, and our approach may be considered for other loss-of function diseases.

### SOD1 Metal Content Plays a Role in Stabilization of SOD1

The metal contents of as-isolated WT, G93A, and G85R SOD1 were determined using inductively coupled plasma mass spectrometry (ICP-MS), which are listed in supplemental table 1: as-isolated WT SOD1 contained approximately two molecules of copper and zinc per monomer, as-isolated G93A contained one molecule of copper and one and a half molecules of zinc per monomer, whereas G85R contained less than one copper and one and a half zinc per monomer. From these data it appears that WT and G93A SOD1 were metal replete, and that in addition some adventitious (non-active site) metal binding occurred. We therefore also analyzed metal content using FTMS, where we have observed that the desolvation process tends to remove most adventitious metals: as-isolated WT SOD1 appeared to be fully metallated, as-isolated G93A SOD1 appeared to be ∼95 % metallated, and as-isolated G85R SOD1 appeared to be partially metallated ∼70 % metallated with a larger population of either unmetallated or singly metallated fractions. These data may explain the increase in stability observed when exogenous metals were added to the G85R sample versus the G93A sample, because the population of G85R consists of a larger percentage of partially metallated and unmetallated forms.

### EQUIVALENTS

Those skilled in the art will recognize, or be able to ascertain using no more than routine experimentation, many equivalents to the specific embodiments of the invention described herein.

## Claims

1. A stabilized superoxide dismutase analogue, wherein said analogue has a tertiary structure and comprises a first SOD1 monomer, a second SOD1 monomer, and a crosslinker; said crosslinker comprises a spacer arm with length from 3 Å to 15 Å; wherein said first SOD1 monomer comprises a first cysteine at a first position corresponding to position 111 of wild-type human SOD1, and said second SOD1 monomer comprises a second cysteine at a second position corresponding to position 111 of wild-type human SOD1; and the first cysteine is covalently connected to the second cysteine by the crosslinker.

2. The analogue of claim 1, wherein any one or more of the following applies,
a) the tertiary structure is substantially the same as the wild-type human superoxide dismutase enzyme;
b) the first cysteine and the second cysteine occupy the same numbered position;
c) the first cysteine and the second cysteine occupy different numbered positions;
d) the first cysteine is at position 111; and
e) the second cysteine is at position 111.

3. The analogue of claims 1 or 2, wherein the sequence identity of said first SOD1 monomer and said second SOD1 monomer is greater than or equal to 85%; and/or said first SOD1 monomer and said second SOD1 monomer have the same amino acid sequence.

4. The analogue of any one of claims 1-3, wherein the first SOD1 monomer of said analogue is the wild-type human sequence or is a human SOD1 monomer comprising a mutation selected from the group consisting of G93A, G85R, D90A, A4V, E100G, H46R, C6G, and I113T; and/or
the second SOD1 monomer of said analogue is the wild-type human sequence or is a human SOD1 monomer comprising a mutation selected from the group consisting of G93A, G85R, D90A, A4V, E100G, H46R, C6G, and I113T.

5. The analogue of any one of claims 1-4, wherein said analogue retains at least 90% activity of the wild-type human superoxide dismutase enzyme up to a temperature of 75 degrees C.

6. The analogue of any one of claims 1-5, wherein any one of the following applies,
a) the unfolding temperature of said analogue is increased by from 10 degrees C to 60 degrees C as compared to its uncrosslinked dimer;
b) the unfolding temperature of said analogue is increased by from 20 degrees C to 40 degrees C as compared to its uncrosslinked dimer;
c) the unfolding temperature of said analogue is increased by from 15 degrees C to 25 degrees C as compared to its uncrosslinked dimer;
d) the unfolding temperature of said analogue is increased by from 30 degrees C to 50 degrees C as compared to its uncrosslinked dimer;
e) the unfolding temperature of said analogue is increased by 20 degrees C as compared to its uncrosslinked dimer; and
f) the unfolding temperature of said analogue is increased by 40 degrees C as compared to its uncrosslinked dimer.

7. The stabilized superoxide dismutase analogue of any one of claims 1-6, wherein optionally,
a)
i) the crosslinker is selected from the group consisting of DTME, TMEA, BMDB, BM(PEG)₂, BMB, and BMOE; or
ii) the crosslinker is selected from the group consisting of organomercurials, maleimides, vinyl sulfones, and alkylating agents; and/or
b) any one of the following applies,
a) the crosslinker comprises a spacer arm with length from 7 Angstroms to 11 Angstroms;
b) the crosslinker comprises a spacer arm with length of 9 Angstroms.

8. A pharmaceutical composition comprising a stabilized SOD1 analogue of claim 1; and a pharmaceutically acceptable carrier.

9. A method of producing a stabilized superoxide dismutase (SOD1) analogue according to claim 1 comprising,
a) the step of reacting a first SOD1 monomer, a second SOD1 monomer, and a cross-linker, thereby forming said analogue; or
b) the step of connecting by a cross-linker a first cysteine of a first SOD1 monomer with a second cysteine of a second SOD1 monomer.

10. The method of claim 9, wherein any one or more of the following applies,
a) the method further comprising the step of replacing a naturally occurring amino acid of the first SOD1 monomer with the first cysteine; and
b) the method further comprising the step replacing a naturally occurring amino acid of the second SOD1 monomer with the second cysteine.

11. A stabilized SOD1 analogue of any one of claims 1-8 for use in treatment of a neurodegenerative disease or in prophylaxis for neurodegenerative disease, wherein optionally,
i) the neurodegenerative disease is amyotrophic lateral sclerosis; and/or
ii) the mammal is a primate, bovine, ovine, equine, porcine, rodent, feline, or canine; and/or
iii) the mammal is a human.

## Patentansprüche

1. Ein stabilisiertes Superoxid-Dismutase-Analogon, wobei das genannte Analogon eine tertiäre Struktur aufweist und ein erstes SOD1-Monomer, ein zweites SOD1-Monomer und einen Vernetzer umfasst; wobei der genannte Vernetzer einen Spacer einer Länge von 3 Å bis 15 Å umfasst; wobei das genannte erste SOD1-Monomer ein erstes Cystein in einer ersten Position aufweist, die Position 111 eines menschlichen Wildtyp-SOD1-Monomers entspricht, und das genannte zweite SOD1-Monomer ein zweites Cystein in einer zweiten Position umfasst, , die Position 111 eines menschlichen Wildtyp-SOD1-Monomers entspricht; und das erste Cystein durch einen Vernetzer kovalent mit dem zweiten Cystein verbunden ist.

2. Das Analogon entsprechend Anspruch 1, wobei eines oder mehrere der Folgenden zutrifft/zutreffen:
a) die tertiäre Struktur ist wesentlich die selbe wie das menschliche Wildtyp-Superoxid-Dismutase-Enzym;
b) das erste Cystein und das zweite Cystein nehmen die selbe nummerierte Position ein;
c) das erste Cystein und das zweite Cystein nehmen unterschiedliche nummerierte Positionen ein;
d) das erste Cystein befindet sich in Position 111; und
e) das zweite Cystein befindet sich in Position 111.

3. Das Analogon entsprechend Anspruch 1 oder 2, wobei die Sequenzidentität des genannten ersten SOD1-Monomers und des genannten zweiten SOD1-Monomers größer als oder gleich 85% ist; und/oder das genannte erste SOD1-Monomer und das genannte zweite SOD1-Monomer haben die selbe Aminosäuresequenz.

4. Das Analogon entsprechend einem der Ansprüche 1-3, wobei das erste SOD1-Monomer des genannten Analogons die menschliche Wildtyp-Sequenz ist oder ein menschliches SOD1-Monomer ist, das eine Mutation enthält, die aus der Gruppe bestehend aus G93A, G85R, D90A, A4V, E100G, H46R, C6G und I113T ausgewählt wird; und/oder das zweite SOD1-Monomer des genannten Analogons die menschliche Wildtyp-Sequenz ist oder ein menschliches SOD1-Monomer ist, das eine Mutation enthält, die aus der Gruppe bestehend aus G93A, G85R, D90A, A4V, E100G, H46R, C6G und I113T ausgewählt wird.

5. Das Analogon entsprechend einem der Ansprüche 1-4, wobei das genannte Analogon zumindest 90% Aktivität des menschlichen Wildtyp-Superoxid-Dismutase-Enzyms bis zu einer Temperatur von 75°C beibehält.

6. Das Analogon entsprechend einem der Ansprüche 1-5, wobei eines der Folgenden zutrifft;
a) die Entfaltungstemperatur des genannten Analogons wird im Vergleich zu seinem nicht vernetzten Dimer von 10°C auf 60°C erhöht;
b) die Entfaltungstemperatur des genannten Analogons wird im Vergleich zu seinem nicht vernetzten Dimer von 20°C auf 40°C erhöht;
c) die Entfaltungstemperatur des genannten Analogons wird im Vergleich zu seinem nicht vernetzten Dimer von 15°C auf 25°C erhöht;
d) die Entfaltungstemperatur des genannten Analogons wird im Vergleich zu seinem nicht vernetzten Dimer von 30°C auf 50°C erhöht;
e) die Entfaltungstemperatur des genannten Analogons wird im Vergleich zu seinem nicht vernetzten Dimer um 20°C erhöht;
f) die Entfaltungstemperatur des genannten Analogons wird im Vergleich zu seinem nicht vernetzten Dimer um 40°C erhöht.

7. Das stabilisierte Superoxid-Dismutase-Analog entsprechend einem der Ansprüche 1-6, wobei optional,
a)
i) der Vernetzer aus der Gruppe bestehend aus DTME; TMEA, BMDB, BM(PEG)₂, BMB und BMOE ausgewählt wird; oder
ii) der Vernetzer aus der Gruppe bestehend aus Organoquecksilberverbindungen, Maleimiden, Vinylsulfonen und Alkylierungsmitteln ausgewählt wird; und/oder
b) eines der Folgenden zutrifft,
a) der Vernetzer einen Spacer einer Länge von 7 Angström bis 11 Angström umfasst;
b) der Vernetzer einen Spacer einer Länge von 9 Angström umfasst.

8. Eine pharmazeutische Zusammensetzung, die ein stabilisiertes SOD1-Analogon entsprechend Anspruch 1 und einen pharmazeutisch verträglichen Trägerstoff umfasst.

9. Ein Verfahren zur Erzeugung eines stabilisierten Superoxid-Dismutase-(SOD1)-Analogons entsprechend Anspruch 1 bestehend aus
a) dem Schritt der Auslösung einer Reaktion eines ersten SOD1-Monomers, eines zweiten SOD1-Monomers und eines Vernetzers, wodurch das genannte Analogon gebildet wird, oder
b) dem Schritt des Verbindens eines ersten Cysteins eines ersten SOD1-Monomers mit einem zweiten Cystein eines zweiten SOD!-Monomers durch einen Vernetzer.

10. Das Verfahren entsprechend Anspruch 9, wobei eines oder mehre der Folgenden zutrifft/zutreffen,
a) das Verfahren umfasst weiterhin den Schritt des Ersetzens eines natürlich vorkommenden Aminosäure des ersten SOD1-Monomers durch das erste Cystein; und
b) das Verfahren umfasst weiterhin den Schritt des Ersetzens einer natürlich vorkommenden Aminosäure des zweiten SOD1-Monomers durch das zweite Cystein.

11. Ein stabilisiertes SOD1-Analogon entsprechend einem der Ansprüche 1-8 zur Verwendung bei der Behandlung einer neurodegenerativen Erkrankung oder bei der Prophylaxe einer neurodegenerativen Erkrankung, wobei optional,
i) die neurodegenerative Erkrankung amyotrophe Lateralsklerose ist; und/oder
ii) der Säuger ein Primat, ein Rind, ein Schaf, ein Pferd, ein Schwein, ein Nager, eine Katze oder ein Hund ist; und/oder
iii) der Säuger ein Mensch ist.

## Revendications

1. Un analogue de superoxyde dismutase stabilisée, où ledit analogue possède une structure tertiaire et comprend un premier monomère SOD1, un deuxième monomère SOD1 et un agent de réticulation, ledit agent de réticulation comprenant un bras d'espacement d'une longueur de 3 Å à 15 Å, où ledit premier monomère SOD1 comprend une première cystéine au niveau d'une première position correspondant à la position 111 du SOD1 humain de type sauvage, et ledit deuxième monomère SOD1 comprend une deuxième cystéine au niveau d'une deuxième position correspondant à la position 111 du SOD1 humain de type sauvage, et la première cystéine est raccordée de manière covalente à la deuxième cystéine par l'agent de réticulation.

2. L'analogue selon la Revendication 1, où l'une quelconque ou plusieurs des caractéristiques suivantes s'applique :
a) la structure tertiaire est sensiblement la même que celle de l'enzyme de superoxyde dismutase humaine de type sauvage,
b) la première cystéine et la deuxième cystéine occupent la même position numérotée,
c) la première cystéine et la deuxième cystéine occupent des positions numérotées différentes,
d) la première cystéine est à la position 111, et
e) la deuxième cystéine est à la position 111.

3. L'analogue selon les Revendications 1 ou 2, où l'identité de séquence dudit premier monomère SOD1 et dudit deuxième monomère SOD1 est supérieure ou égale à 85%, et/ou ledit premier monomère SOD1 et ledit deuxième monomère SOD1 possèdent la même séquence d'acides aminés.

4. L'analogue selon l'une quelconque des Revendications 1 à 3, où le premier monomère SOD1 dudit analogue est la séquence humaine de type sauvage ou est un monomère SOD1 humain comprenant une mutation sélectionnée dans le groupe se composant de G93A, G85R, D90A, A4V, E100G, H46R, C6G et I113T, et/ou
le deuxième monomère SOD1 dudit analogue est la séquence humaine de type sauvage ou est un monomère SOD1 humain comprenant une mutation sélectionnée dans le groupe se composant de G93A, G85R, D90A, A4V, E100G, H46R, C6G et I113T.

5. L'analogue selon l'une quelconque des Revendications 1 à 4, où ledit analogue conserve au moins 90% d'activité de l'enzyme de superoxyde dismutase humaine de type sauvage jusqu'à une température de 75°C.

6. L'analogue selon l'une quelconque des Revendications 1 à 5, où l'une quelconque des caractéristiques suivantes s'applique,
a) la température de dépliage dudit analogue est augmentée de 10°C à 60°C en comparaison de son dimère non réticulé,
b) la température de dépliage dudit analogue est augmentée de 20°C à 40°C en comparaison de son dimère non réticulé,
c) la température de dépliage dudit analogue est augmentée de 15°C à 25°C en comparaison de son dimère non réticulé,
d) la température de dépliage dudit analogue est augmentée de 30°C à 50°C en comparaison de son dimère non réticulé,
e) la température de dépliage dudit analogue est augmentée de 20°C en comparaison de son dimère non réticulé, et
f) la température de dépliage dudit analogue est augmentée de 40°C en comparaison de son dimère non réticulé.

7. L'analogue de superoxyde dismutase stabilisée selon l'une quelconque des Revendications 1 à 6, où éventuellement,
a)
i) l'agent de réticulation est sélectionné dans le groupe se composant de DTME, TMEA, BMDB, BM(PEG)₂, BMB et BMOE, ou
ii) l'agent de réticulation est sélectionné dans le groupe se composant d'organomercuriels, de maléimides, de sulfones de vinyle et d'agents d'alkylation, et/ou
b) l'une quelconque des caractéristiques suivantes s'applique,
i) l'agent de réticulation comprend un bras d'espacement d'une longueur de 7 Å à 11 Å,
ii) l'agent de réticulation comprend une bras d'espacement d'une longueur de 9 Å.

8. Une composition pharmaceutique contenant un analogue de SOD1 stabilisée selon la Revendication 1 et un vecteur pharmaceutiquement acceptable.

9. Un procédé de production d'un analogue de superoxyde dismutase stabilisée (SOD1) selon la Revendication 1 comprenant :
a) l'opération de réaction d'un premier monomère SOD1, d'un deuxième monomère SOD1 et d'un agent de réticulation, formant ainsi ledit analogue, ou
b) l'opération de raccordement par un agent de réticulation d'une première cystéine d'un premier monomère SOD1 à une deuxième cystéine d'un deuxième monomère SOD1.

10. Le procédé selon la Revendication 9, où l'une quelconque ou plusieurs des caractéristiques suivantes s'applique :
a) le procédé comprenant en outre l'opération de remplacement d'un acide aminé naturel du premier monomère SOD1 par la première cystéine, et
b) le procédé comprenant en outre l'opération de remplacement d'un acide aminé naturel du deuxième monomère SOD1 par la deuxième cystéine.

11. Un analogue de SOD1 stabilisée selon l'une quelconque des Revendications 1 à 8 destiné à une utilisation dans le traitement d'une maladie neurodégénérative ou dans la prophylaxie d'une maladie neurodégénérative, où éventuellement,
i) la maladie neurodégénérative est la sclérose latérale amyotrophique, et/ou
ii) le mammifère est un primate, bovin, ovin, équidé, porcidé, rongeur, félin ou canidé, et/ou
iii) le mammifère est un humain.
